# EUROPÄISCHE PATENTANMELDUNG

(11) **EP 3 168 762 A1**
(43) Veröffentlichungstag der Anmeldung: **17.05.2017**
(21) Anmeldenummer: 16020444.2
(22) Anmeldetag: 10.11.2016
(51) Int. Cl.: G06F 19/00

(54) **VERFAHREN UND SYSTEM ZUR INFORMATIONSTECHNISCHEN UNTERSTÜTZUNG BEI EINEM THERAPIEVERLAUF**

(30) Priorität: 13.11.2015 DE 102015014603
(71) Anmelder: Röder, Carsten, 59519 Möhnesee (DE); Lehnert, Oliver, 59494 Soest (DE)
(72) Erfinder: Röder, Carsten, 59519 Möhnesee (DE); Lehnert, Oliver, 59494 Soest (DE)
(74) Vertreter: Von Rohr Patentanwälte Partnerschaft mbB

(57) **Zusammenfassung**

Die Erfindung betrifft ein Verfahren zur informationstechnischen Unterstützung bei einem Therapieablauf für einen Patienten, wobei eine Ausgabe durch eine Benutzerschnittstelle (7) auf Basis mindestens zweier zu unterschiedlichen Therapiestationen (T₁, T₂) korrespondierender, patientenspezifische und/oder therapierelevante Parameter aufweisender Datensätze (2A, 2B) erfolgt. Ferner betrifft die Erfindung ein System zur informationstechnischen Unterstützung bei einem Therapieablauf und die Verwendung des Verfahrens und/oder des Systems.

## Beschreibung

Die vorliegende Erfindung betrifft ein Verfahren, ein System und eine Verwendung zur informationstechnischen Unterstützung bei einem Therapieablauf für einen Patienten.

Im Gesundheitswesen wird zunehmend Gebrauch von der informationstechnologischen Entwicklung gemacht. Durch elektronische Unterstützung werden die vielschichtigen und zum Teil äußerst komplexen Vorgänge in einer medizinischen bzw. therapeutischen Einrichtung, beispielsweise einem Krankenhaus oder einer stationären Rehabilitations-Einrichtung, erheblich vereinfacht. Zum einen wird durch zunehmende Vernetzung die Kommunikation beschleunigt. Zum anderen ermöglicht beispielsweise die Dokumentation eines Behandlungsverlaufs in einer elektronischen Krankenakte einen schnelleren und einfacheren Zugriff auf die darin gespeicherten Daten. Ferner können wesentliche Informationen leichter strukturiert werden.

Eine elektronische Krankenakte ist häufig in ein einrichtungsweites Krankenhausinformationssystem (KIS) eingebunden. Ferner existieren im Gesundheitswesen beispielsweise Systeme für ein Ressourcenmanagement, wie sie aus dem Logistikbereich bekannt sind.

Innerhalb einer Einrichtung erleichtern Systeme der vorgenannten Art dem medizinisch-therapeutischen Personal wie Pflegekräften und Ärzten organisatorische Abläufe, die mit der medizinischen Versorgung in Verbindung stehen. Bekannte Systeme sind jedoch auf eine konkrete medizinische Einrichtung zugeschnitten und lokal begrenzt. Bei einem Standortwechsel des Patienten in eine andere medizinische und/oder therapeutische Einrichtung kommt es daher zu Medienbrüchen und infolge dessen zu verwaltungstechnischem Mehraufwand, Doppeluntersuchungen sowie zu Verzögerungen und Fehlentscheidungen bezüglich des Therapieablaufs.

So beschränken sich die zur Weiterbehandlung zur Verfügung stehenden Informationen derzeit auf einen Entlassungsbericht der vorhergehenden Einrichtung. Selbst wenn dieser bereits vor dem Antritt der Weiterbehandlung des Patienten bei der betreffenden Einrichtung vorliegt, sind häufig dennoch weitere Untersuchungen nach der Aufnahme des Patienten am neuen Standort notwendig, um die Weiterbehandlung, beispielsweise in Form von Rehabilitations-Maßnahmen, in geeigneter Weise durchzuführen oder um bestimmte Pflegebedürfnisse zu erfüllen. Es kommt dabei häufig zu Doppeluntersuchungen, die kostenintensiv sind und darüber hinaus auch eine körperliche und/oder psychische Belastung für die betroffenen Patienten darstellen. Ferner können erhebliche Wartezeiten im gesamten Therapieablauf eines Patienten auftreten, beispielsweise zwischen dem Aufenthalt in einer Akutklinik und der Weiterbehandlung in einer Rehabilitations-Einrichtung, da zunächst eine Einrichtung ausgewählt und am entsprechenden Standort ein freier Behandlungsplatz zur Verfügung stehen muss. Solche Wartezeiten gefährden das Gesamtergebnis, da sich das Therapieergebnis während dieser Wartezeit verschlechtern kann.

Vor diesem Hintergrund ist es Aufgabe der vorliegenden Erfindung, ein Verfahren und ein System sowie eine Verwendung anzugeben, wodurch ein Therapieablauf verbessert werden kann, insbesondere so dass eine Therapie effizienter, effektiver, schneller und/oder reproduzierbarer wird.

Die vorgenannte Aufgabe wird durch ein Verfahren mit den Merkmalen des Patentanspruchs 1, ein System mit den Merkmalen des Patentanspruchs 7 oder durch eine Verwendung mit den Merkmalen des Patentanspruchs 10 gelöst. Vorteilhafte Weiterbildungen sind Gegenstand der Unteransprüche.

Ein erster Aspekt der vorliegenden Erfindung betrifft ein Verfahren zur informationstechnischen Unterstützung bei einem Therapieablauf für einen Patienten. In diesem Verfahren erfolgt eine Ausgabe durch eine Benutzerschnittstelle auf Basis mindestens zweier, zu unterschiedlichen Therapiestationen korrespondierender, patientenspezifische und/oder therapierelevante Parameter aufweisender Datensätze.

Es hat sich in überraschender Weise gezeigt, dass eine informationstechnisch basierte Unterstützung, insbesondere Führung, des Therapieablaufs zu einem verbesserten Therapieergebnis führt, da Wartezeiten mit damit einhergehenden potenziellen Verschlechterungen des Therapieergebnisses vermieden werden. Zudem ermöglicht die informationstechnisch basierte bzw. automatische Unterstützung, insbesondere Führung, des Therapieablaufs bzw. durch den Therapieablauf, vorzugsweise mittels der Ausgabe, eine verbesserte Auslastung von Ressourcen wie Geräten und Personal.

### Definitionen

Ein Therapieablauf im Sinne der vorliegenden Erfindung ist vorzugsweise eine, insbesondere chronologische, Aneinanderreihung mehrerer Therapiestationen. Insbesondere handelt es sich also um eine Abfolge von Untersuchungen und Therapien bzw. Therapiemaßnahmen. Der Therapieablauf kann mittels der vorliegenden Erfindung optimiert werden, wodurch ein schnelleres bzw. besseres Therapieergebnis erreicht werden kann.

Ein Patient im Sinne der vorliegenden Erfindung ist vorzugsweise ein Lebewesen, insbesondere Mensch, der ärztliche Dienstleistungen oder Dienstleistungen anderer Personen, die eine Heilbehandlung durchführen, in Anspruch nimmt. Die Heilbehandlung kann der Linderung von Krankheiten oder Folgen eines Unfalls dienen, an denen der Patient leidet, oder sonstige Maßnahmen die medizinische Behandlungen erfordern oder umfassen, insbesondere einschließlich schönheitschirurgischer Maßnahmen. Zur Personengruppe der Patienten könne also auch gesunde Lebewesen gehören.

Eine Ausgabe im Sinne der vorliegenden Erfindung kann vorzugsweise eine grafische Ausgabe auf einem Anzeigegerät bzw. einer grafischen Benutzerschnittstelle - GUI - sein oder aufweisen. Eine Ausgabe im Sinne der vorliegenden Erfindung kann jedoch alternativ oder zusätzlich die Bereitstellung von Parametern in maschinenlesbarer Form, beispielsweise an oder für medizinisch-diagnostisches Gerät, insbesondere dessen Schnittstelle für Konfigurationsdaten, sein oder aufweisen. Die Ausgabe kann Therapiestation oder ein Teil hiervon, ein oder mehrere hierzu korrespondierende Parameter, ein Datensatz oder Teil hiervon und/oder eine Arbeitsanweisung und/oder Handlungsempfehlung an einen Benutzer umfassen.

Eine Benutzerschnittstelle im Sinne der vorliegenden Erfindung ist bevorzugt dazu ausgebildet, Informationen zur Verfügung zu stellen, zu senden und/oder zu empfangen. Besonders bevorzugt handelt es sich um eine Schnittstelle zur visuellen Ausgabe, insbesondere Darstellung auf einem Display, einer elektronischen Schautafel o.dgl. Die Benutzerschnittstelle weist vorzugsweise Anzeigegerät bzw. einer grafischen Benutzerschnittstelle - GUI - auf oder ist hierdurch gebildet. Alternativ oder zusätzlich weist die Benutzerschnittstelle eine Eingabeeinrichtung bzw. ein Eingabegerät auf, womit vorzugsweise Auswahlen getroffen und/oder Parameter eingegeben werden können.

Die Benutzerschnittstelle kann ein Tablet-Computer, ein Laptop, ein Smartphone oder ein sonstiges Eingabe- und/oder Anzeigegerät sein oder aufweisen. Die Benutzerschnittstelle kann jedoch auch anderweitig gebildet sein und/oder zur Informationsübertragung an ein Gerät, insbesondere Medizingerät, ausgebildet sein. Die Benutzerschnittstelle kann auch mehrere Anzeigegeräte und/oder Eingabegeräte, insbesondere mehrere Geräte aus der Gruppe der Tablet-Computer, ein Laptops, ein Smartphones und/oder elektronischen Schautafeln bzw. Displays sein oder aufweisen.

In einer Ausführungsform ist die Benutzerschnittstelle ein persönlicher digitaler Assisten oder die Benutzerschnittstelle weist einen solchen auf. Hierbei kann es sich um eine Systemkomponente handeln. Vorzugsweise ist der persönliche digitale Assist ein tragbares Gerät wie ein Patientenarmband.

Mit dem persönlichen digitalen Assistenten kann vorzugsweise vollautomatisch an Termine erinnert werden. Alternativ oder zusätzlich können Zielorte oder nächste Schritte ausgegeben oder angezeigt werden und/oder Parameter, insbesondere Vitalfunktionsbezogene Werte, durch den persönlichen digitalen Assistenten automatisch ermittelt, eingelesen, eingegeben, übermittelt und/oder als Parameter der Datensätze bzw. für das vorschlagsgemäße Verfahren verwendet werden.

Auf diese oder sonstige Weise erfolgt vorzugsweise eine Benutzerführung mit dem persönlichen digitalen Assistenten. Die Benutzerführung kann jedoch auch durch sonstige Benutzerschnittstellen und/oder in anderer Weise durch die vorschlagsgemäße Ausgabe erfolgen. Die Benutzerführung erfolgt vorzugsweise basierend auf den Parametern, Datensätzen und Therapiestationen, insbesondere mit dem vorschlagsgemäßen Verfahren.

Bei einem Benutzer bzw. Nutzer im Sinne der vorliegenden Erfindung handelt es sich insbesondere um eine im medizinisch-therapeutischen Bereich tätige Person und/oder einen Patienten. Es kommen vorzugsweise jedoch auch andere, beispielsweise technische Benutzer bzw. Nutzer wie Automaten, Roboter oder dergleichen in Frage.

Dementsprechend ist die Benutzerschnittstelle vorzugsweise nicht auf die Interaktion mit einem insbesondere menschlichen Benutzer beschränkt. Die Benutzerschnittstelle stellt als Schnittstelle vorzugsweise einen allgemeinen Übergabepunkt zur Eingabe und/oder Ausgabe von Parametern dar. Im Speziellen ist jedoch bevorzugt, dass die Benutzerschnittstelle jedenfalls eine Kommunikation bzw. Interaktion zwischen System und menschlichen Benutzer ermöglicht.

Eine Therapiestation im Sinne der vorliegenden Erfindung korrespondiert vorzugsweise zu einer, bevorzugt diagnostischen und/oder therapeutischen, Maßnahme. Eine Therapiestation weist vorzugsweise eine räumliche, zeitliche und/oder funktionale Gegebenheit / Maßnahme oder eine Kombination oder ein Satz hieraus auf oder ist hieraus oder hiermit gebildet. Bei einer Therapiestation handelt es sich vorzugsweise um einen funktionalen Abschnitt eines Therapieablaufs, der vorzugsweise einer, insbesondere zeitlich, örtlich und/oder funktional unmittelbar zusammenhängenden Maßnahme zugeordnet ist.

Eine Therapiestation ist technisch vorzugsweise durch einen oder mehrere Parameter bzw. einen oder mehrere Datensätze aufweisend solche Parameter repräsentiert. Insbesondere korrespondiert zu einer Therapiestation genau ein Datensatz. Dieser kann zusammenhängend oder mehrteilig, insbesondere auch über mehrere Orte verteilt sein. Ein Datensatz liegt vorzugsweise dann vor, wenn Daten, insbesondere Parameter, durch Zuordnung zu derselben Therapiestation miteinander in Beziehung stehen.

Unterschiedliche Datensätze sind vorzugsweise jeweils dadurch charakterisiert, dass die Parameter der Datensätze zu unterschiedlichen Therapiestationen korrespondieren. Ein erster Datensatz kann hierbei zu einer ersten Therapiestation korrespondieren, ein zweiter Datensatz kann zu einer zweiten Therapiestation korrespondieren und so weiter.

Verschiedene Therapiestationen unterscheiden sich vorzugsweise dadurch, dass sie zu unterschiedlichen Maßnahmen, Zeitpunkten von Maßnahmen und/oder Orten von Maßnahmen korrespondieren. Eine erste Therapiestation weicht also vorzugsweise jedenfalls hinsichtlich der Maßnahme, des Zeitpunkts / Termins oder des Orts von einer zweiten oder weiteren Therapiestation ab. Hierbei kann eine Abweichung in einer der Kategorien ausreichen.

Die eine Therapiestation charakterisierenden Orts- und Zeitdaten sowie Daten, die im Zusammenhang mit bei der Therapiestation durchzuführenden diagnostischen und/oder therapeutischen Maßnahmen stehen, werden vorzugsweise durch die entsprechenden Parameter im zu der betreffenden Therapiestation korrespondierenden Datensatz abgebildet oder repräsentiert.

Grundsätzlich können sich unterschiedliche Therapiestationen auch in nahezu allen Punkten gleichen und beispielsweise nur im zugehörigen Zeitpunkt und/oder Ort der zugehörigen Maßnahme unterscheiden, wie es bei Wiederholungsuntersuchungen zur Verifikation von Untersuchungsergebnissen oder zur Ermittlung einer zeitlichen Entwicklung von gemessenen Werten der Fall ist.

Die Parameter bzw. Datensätze korrespondieren insbesondere zu und/oder repräsentieren insbesondere technischen Gegebenheiten, technischen Maßnahmen, Geräte, Geräteinstellungen, Konzentrationen, Volumina, Koordinaten, Orte, Zeiten und/oder Ressourcen, jeweils vorzugsweise im Zusammenhang mit einer Therapiestation.

Die in einem Datensatz enthaltenen Parameter charakterisieren bzw. definieren vorzugsweise eine Therapiestation und stehen insbesondere im Zusammenhang mit dem Ort, der Zeit und/oder diagnostischen und/oder therapeutischen Maßnahmen in Verbindung mit der Therapiestation. Ein zu der Therapiestation korrespondierender Datensatz ist also ein Satz dieser die Therapiestation charakterisierenden Parameter.

Ein Datensatz muss dabei allerdings nicht zwingend einen vollständigen, d. h. zur genauen Definition bzw. Charakterisierung einer Therapiestation notwendigen, Parametersatz umfassen. Der Datensatz kann ein oder mehrere zur Füllung mit Parametern vorgesehene Felder aufweisen, die zunächst unbesetzt sind, d. h. keinen entsprechenden Parameter aufweisen.

Grundsätzlich kann eine Therapiestation auch lediglich durch wenigstens eine mit ihr in Verbindung stehende Maßnahme charakterisiert sein. Unterschiedliche Therapiestationen unterscheiden sich dementsprechend insbesondere in den durchzuführenden diagnostischen und/oder therapeutischen Maßnahmen.

Der zu einer lediglich durch wenigstens eine Maßnahme definierten Therapiestation korrespondierende Datensatz weist insbesondere nur einen oder mehrere Parameter betreffend diese Maßnahme bzw. Maßnahmen auf. Beispielsweise den Ort und/oder die Zeit einer Therapiemaßnahme betreffende Parameter können dem Datensatz ergänzend zu einem späteren Zeitpunkt hinzugefügt werden. Insbesondere wird ein Ressourcenbedarf im Zusammenhang mit der Therapiestation in der Regel durch eine mit der Therapiestation in Verbindung stehende Maßnahme bedingt bzw. definiert. Aus einem Abgleich des Ressourcenbedarfs mit einer gegebenen Ressourcenverfügbarkeit, vorzugsweise mittels eines Ressourcenmanagementsystems, können sich Parameter bezüglich eines möglichen Ortes und/oder einer möglichen Zeit zur Durchführung der betreffenden Maßnahme ergeben. Diese Parameter können die unbesetzten Felder eines in der vorgenannten Art unvollständigen Datensatzes besetzen. Der auf diese Weise vervollständigte Datensatz bildet parameterseitig nun exakt die zu ihm korrespondierende Therapiestation in Bezug auf den Ort, die Zeit und durchzuführende Maßnahmen ab.

Ferner kann ein Datensatz auch mehrteilig ausgebildet sein. D. h. ein Datensatz muss keine statische, zusammenhängende Gruppierung einer Mehrzahl von Daten bzw. Parametern sein. Ein Datensatz kann insbesondere auch durch wenigstens ein dynamisches Element, wie einen Link, einen Pointer oder allgemein einen Verweis, einen oder mehrere Parameter anderer Datensätze oder allgemein beliebiger Datenquellen miteinbeziehen. Derart referenzierte Parameter werden bei dem erfindungsgemäßen Verfahren vorzugsweise in gleicher Weise verwertet bzw. verarbeitet wie die im Datensatz selbst enthaltenen Parameter.

Alternativ oder zusätzlich repräsentieren die Parameter bzw. Datensätze und/oder korrespondieren zu bei oder im Zusammenhang mit der Gegebenheit bzw. Maßnahme ermittelten Parametern, Vitalparametern, Untersuchungsergebnissen, Therapieergebnissen o.dgl.

Insbesondere handelt es sich bei den Parametern um Zahlenwerte, die vorzugsweise mit Einheiten versehen und/oder bestimmten Gegebenheiten und/oder Funktionen zugeordnet sind. Die Parameter können jedoch auch sonstige Indikatoren, alphanumerische Zeichenketten o.dgl. sein oder aufweisen.

Eine Phase des Therapieablaufs ist vorzugsweise ein, bevorzugt funktional und/oder zeitlich, zusammenhängender Abschnitt des Therapieablaufs. Eine Phase weist hierbei vorzugsweise eine oder mehrere Therapiestationen auf.

So können Phasen des Therapieablaufs, beispielsweise der Aufenthalt in einer Akutklinik und der nachfolgende Aufenthalt in einer Rehabilitationseinrichtung an verschiedenen geografischen Standorten, verschiedenen Räumen innerhalb eines Gebäudekomplexes oder Geländes oder verschiedenen Einrichtungen wie Krankenhäusern oder Rehabilitationszentren, jeweils unterschiedliche Therapiestationen und/oder Phasen mit unterschiedliche Therapiestationen darstellen.

Vorzugsweise werden Ereignisse oder Maßnahmen, die als Teilaspekte einer Phase zu verschiedenen Zeitpunkten in derselben oder unterschiedlichen Einrichtung erfolgen, als einzelne Therapiestationen definiert. Beispiele hierfür sind etwa die Aufnahme und Anamnese des Patienten zu Beginn der Behandlung und eine später stattfindende Untersuchung.

Bei therapierelevanten Parametern kann es sich um diagnostische und/oder kurative bzw. therapeutische Parameter handeln. Therapierelevante Parameter betreffen vorzugsweise die Maßnahme der jeweiligen Therapiestation, insbesondere inhaltlich.

Bei diagnostischen Parametern kann es sich neben zahlenmäßig erfassten oder zu erfassenden Werten wie Vitalparametern beispielsweise auch um erfasste oder zu erfassende Daten in Form von Graphen, Grafiken, von Berichten zu Untersuchungen, Operationen, Bilddaten diagnostischer Verfahren oder dgl. handeln.

Bei therapierelevanten kurativen bzw. therapeutischen Parametern kann es sich um Einstellungen für technische Geräte, insbesondere Medizingeräte, und/oder um Parameter durchzuführender Maßnahmen, Ablaufschemata, Maßnahmenkataloge o.dgl. handeln.

Vorzugsweise sind ein oder mehrere organisatorische Parameter vorgesehen. Vorzugsweise weist ein oder mehrere der Datensätze (jeweils) ein oder mehrere organisatorische Parameter auf. Die organisatorischen Parameter sind vorzugsweise jeweils Therapiestationen zugeordnet oder korrespondieren hierzu oder umgekehrt. Organisatorische Parameter korrespondieren vorzugsweise zu organisatorischen Angaben, insbesondere Orten, Zeiten oder dergleichen.

Es kann auch zwischen (diagnostischen und/oder kurativen bzw. therapeutischen und/oder organisatorischen) Durchführungsparametern und (diagnostischen und/oder kurativen bzw. therapeutischen und/oder organisatorischen) Ergebnisparametern differenziert werden, wobei Durchführungsparameter Maßnahmen und Ergebnisparameter die erzielten und/oder zu erzielenden Ergebnisse repräsentieren.

Ein diagnostischer Durchführungsparameter kann beispielsweise eine Kombination von Einstellungen für die Richtung und Strahlungsintensität zur Erstellung eines Röntgenbilds und eine genaue Lage eines Patienten sein, während ein diagnostischer Ergebnisparameter eine wertemäßige Repräsentation des Röntgenbilds ist oder aufweist.

Ein therapeutischer Durchführungsparameter kann Details einer geplanten Schnittführung sein. Ein therapeutischer Ergebnisparameter kann ein Parameter betreffend eine erfolgreiche chirurgische Entfernung sein.

Es können jedoch auch sonstige, beispielsweise organisatorische, Durchführungsparameter und/oder Ergebnisparameter vorgesehen sein. Ein organisatorischer Durchführungsparameter ist beispielsweise eine Raumnummer, ein Termin o.dgl.

Alternativ oder zusätzlich weist einer oder weisen mehrere der Datensätze übergeordnete patientenspezifische bzw. personenspezifische Parameter, wie beispielsweise Name, Geburtsdatum, Wohnort oder Versichertenstatus, auf.

Möglich ist zudem auch eine Kombination aus patientenspezifischen und therapierelevanten und/oder sonstigen der Parameter in einem Datensatz.

### Gemeinsame Verarbeitung

Die Ausgabe durch eine Benutzerschnittstelle erfolgt vorzugsweise durch gemeinsame Verarbeitung der mindestens zwei zu unterschiedlichen Therapiestationen korrespondierenden Datensätze bzw. den, insbesondere patientenspezifischen und/oder therapierelevanten, Parameter dieser. Es handelt sich vorzugsweise um Parameter, insbesondere Ergebnisparameter, bereits erfolgter bzw. abgeschlossener Therapiestationen.

Vorzugsweise werden der Ausgabe durch die Benutzerschnittstelle mindestens zwei Datensätze zugrunde gelegt, wobei die Datensätze zu unterschiedlichen Therapiestationen korrespondieren.

Gemäß einem Aspekt der vorliegenden Erfindung wird/werden mit den Datensätzen eine oder mehrere potenzielle nächste oder zukünftige bzw. für den weiteren Therapieablauf geeignete Therapiestationen und/oder Einzelheiten bzw. Eigenschaften dieser ermittelt.

Bevorzugt wird auf einen oder mehrere Datensätze ein Verfahren oder ein Algorithmus angewendet, um wenigstens einen neuen Datensatz zu erzeugen. Der neue Datensatz korrespondiert vorzugsweise zu einer weiteren, insbesondere einer dritten, d. h. zur ersten und zweiten Therapiestation unterschiedlichen, Therapiestation.

Bevorzugt werden Ergebnisparameter mehrerer unterschiedlicher Therapiestationen gemeinsam verarbeitet, um einen oder mehrere Durchführungsparameter einer oder mehrerer folgender Therapiestationen zu ermitteln. Insbesondere werden Durchführungsparameter durch Parametervergleiche, Sollwert-Istwert-Vergleiche, Schwellwertbildung und/oder Wichtungen, vorzugsweise unter Verwendung von Referenzwerten, insbesondere aus Referenzwertdatenbanken, ermittelt.

Vorzugsweise werden in einem ersten Schritt therapeutische und/oder diagnostische Durchführungsparameter und auf Basis dieser organisatorische Durchführungsparameter ermittelt. Hier sind jedoch auch andere Lösungen möglich.

Der neue bzw. dritte Datensatz kann wenigstens eine Eigenschaft, insbesondere der Maßnahme, der weiteren bzw. dritten Therapiestation umfassen und/oder dazu verwendet werden, wenigstens eine Eigenschaft, insbesondere der Maßnahme, der weiteren bzw. dritten Therapiestation zu ermitteln. Bei dieser Eigenschaft handelt es sich insbesondere um einen Ressourcenbedarf oder eine Ressource, beispielsweise ein Medizingerät, ein Medikament oder Zeit von medizinischem Personal wie Pflegern oder Ärzten. Die Eigenschaft kann als Parameter Teil des Datensatzes bilden.

Vorzugsweise wird wenigstens eine Maßnahme zur Vorbereitung der weiteren, insbesondere dritten, Therapiestation initiiert. Dies erfolgt vorzugsweise automatisch. Dadurch werden die Abläufe in der medizinisch-therapeutischen Einrichtung weiter vereinfacht, da die Durchführung bestimmter Tätigkeiten oder zu treffende Entscheidungen für den Benutzer des Verfahrens bzw. Systems, d. h. insbesondere für das medizinisch-therapeutische Personal vorbereitet werden oder gar ohne weiteres Zutun eines Benutzers erfolgen.

In einer vorteilhaften Weiterbildung der Erfindung werden mehr als zwei Datensätze verwendet bzw. gemeinsam verarbeitet, um eine weitere Therapiestation, hierzu korrespondierende Parameter und/oder einen hierzu korrespondierenden Datensatz zu ermitteln.

Beispielsweise werden Ergebnisparameter unterschiedlicher vorheriger Therapiestationen gemeinsam mit einem Ressourcen und/oder Ressourcenverfügbarkeiten repräsentierenden Datensatz gemeinsam verarbeitet, um eine weitere Therapiestation zu ermitteln, die verfügbar ist, wobei nicht verfügbare Therapiestationen verworfen oder ausgefiltert werden können.

In einem weiteren Beispiel werden mehr als zwei frühere bzw. bereits erfolgte Therapiestationen zur Ermittlung einer weiteren, folgenden Therapiestation verwendet. Hierbei können Parameter, insbesondere Ergebnisparameter, aus Datensätzen von mehr als zwei unterschiedlichen, insbesondere früheren bzw. bereits erfolgten Therapiestationen (gemeinsam) verwendet werden.

Es ist möglich, das nicht unmittelbar zu einer Therapiestation korrespondierende Parameter alternativ oder zusätzlich verwendet werden, um die weitere Therapiestation zu ermitteln.

Weiter ist es bevorzugt, dass in der beschriebenen oder auf sonstige Weise mehrere mögliche weitere Therapiestationen ermittelt werden, zwischen denen eine Auswahl erfolgen kann. Hierzu können Therapiestationen Optionen bilden, also Optionen ausgegeben und/oder als Optionen gerankt, priorisiert, freigegeben, konkretisiert und/oder ausgewählt werden. Hierauf wird später noch detaillierter eingegangen.

### Ressourcenermittlung

Vorzugsweise wird eine Verfügbarkeit der für die ermittelte(n) Therapiestation(en) benötigten Ressourcen ermittelt. Hierzu kann, beispielsweise durch Datenbankabfragen und/oder -abgleiche, festgestellt werden, zu welchen potenziell nächsten Therapiestationen benötigte Ressourcen grundsätzlich, wann, mit welcher Wartezeit, in welchem räumlichen Abstand, unter welchen Randbedingungen und/oder zu welchen Konditionen verfügbar sind.

Der Ressourcenbedarf kann mit einer Ressourcendatenbank abgeglichen werden, um eine Verfügbarkeit der jeweiligen ermittelten Therapiestation zu bestimmen. Vorzugsweise erfolgt eine Ausgabe der jeweiligen Therapiestation bzw. hierzu korrespondierender Parameter nur bei positivem Ergebnis, also der Ermittlung einer grundsätzlichen oder konkreten Verfügbarkeit der Ressourcen zur Durchführung der Therapiestation.

### Kenngrößen

Anhand eines oder mehrerer Datensätze kann eine Kenngröße ermittelt und/oder ausgegeben werden. Besonders bevorzugt wird die Kenngröße aus (Ergebnis-) Parametern mehrerer, zu unterschiedlichen Therapiestationen korrespondierender Datensätze bestimmt, insbesondere berechnet.

Vorzugsweise wird die Kenngröße automatisch ermittelt, beispielsweise durch einen Server unter Zugriff auf die Datensätze.

Die Kenngröße kann auch zu mehreren möglichen nächsten Therapiestationen korrespondieren.

Einer Therapiestation bzw. Option können eine oder mehrere Kenngrößen zugeordnet sein.

Die Kenngröße kann einteilig oder mehrteilig sein, die Kenngröße kann also auch unterschiedliche Werte oder Indikatoren aufweisen.

Bei der Kenngröße kann es sich um einen Ressourcenbedarf handeln oder einen solchen aufweisen, insbesondere für eine potenziell folgende bzw. weitere oder ermittelte Therapiestation.

Für eine weitere Therapiestation erforderliche Ressourcen können auf diese Weise bereits im Vorhinein ermittelt werden. Die mit dem Ressourcenbedarf in Verbindung stehende Kenngröße kann mit Daten eines Ressourcenmanagementsystems in Bezug gesetzt werden.

So ist es möglich, dass die Kenngröße einen Bedarf solcher Ressourcen repräsentiert, die auch grundsätzlich, innerhalb eines bestimmten Zeitraums, räumlichen Umkreises o.dgl. verfügbar sind oder sein können.

Alternativ oder zusätzlich repräsentiert die Kenngröße Erfolgsaussichten durch Nutzung bestimmter Therapiestationen.

Die Kenngröße kann darüber hinaus auch mit der Qualität der Behandlung bzw. mit dem Therapieerfolg in Verbindung stehen und zur Qualitätssicherung herangezogen werden.

Ferner kann die Kenngröße ein ökonomischer Indikator einer Therapiestation oder einer Abfolge von Therapiestationen und/oder einzelner Phasen des Therapieablaufs, beispielsweise in Bezug auf die Kosten der Behandlung bzw. Therapie eines Patienten oder von Teilen der Behandlung, sein.

Bevorzugt wird die Kenngröße über die Benutzerschnittstelle wiedergegeben oder ausgegeben, insbesondere in Verbindung mit und/oder zugeordnet zu einer hierzu korrespondierenden Therapiestation, Option für eine Therapiestation, Phase etc.

Besonders geeignet ist eine Darstellung, die nicht den bloßen Daten- bzw. Zahlenwert einer solchen Kenngröße darstellt, sondern die Kenngröße nach einer entsprechenden Klassifizierung etwa in Form eines Piktogramms, Graphs, Charts o.dgl. wiedergibt.

Vorzugsweise kann die Kenngröße oder ein Indikator der Kenngröße in Form einer Ampel oder ampelartigen Darstellung durch oder über die Benutzerschnittstelle dargestellt oder auf sonstige Weise ausgegeben werden. Hierbei wird insbesondere grafisch, farblich und/oder durch Strukturen die Kenngröße als solche oder eine Eigenschaft der Kenngröße, Über- bzw. Unterschreitung einer Schwelle o. dgl. repräsentiert.

Bei der ampelartigen Darstellung handelt es sich insbesondere um ein Symbol mit drei unterschiedlich und untereinander angeordneten grafischen Positionen, die alternativ oder in Kombination aktivierbar sind, um unterschiedliche Zustände zu repräsentieren. Beispielsweise ist es möglich, in entsprechender Weise als Kenngröße einen Indikator für einen Nutzen, Aufwand o. dgl. darzustellen. Alternativ oder zusätzlich kann in einem Therapieablauf bzw. für oder im Vorfeld einer Therapiestation ampelartig über die Benutzerschnittstelle ausgegeben werden, dass der aktuelle Verlauf der Therapie bzw. Therapiestation plangemäß, grenzfällig plangemäß oder nicht mehr plangemäß verläuft. Auf diese Weise ermöglicht die Erfindung eine einfache und effiziente visuelle Kontrolle und vereinfachte bzw. verbesserte Führung des Benutzers.

Durch Simulation einer zukünftigen Entwicklung der Kenngröße, die vorzugsweise anhand eines oder mehrerer Datensätze erfolgt, lassen sich insbesondere Aussagen über einen zu erwartenden Therapieerfolg, erforderliche Therapiemaßnahmen an zukünftigen Therapiestationen und/oder im weiteren Verlauf benötigte Ressourcen im Zusammenhang mit weiteren Therapiestationen treffen. Auch diese können Kenngrößen oder Indikatoren sein.

Ferner kann durch die Simulation der Entwicklung der Kenngröße auch eine Abschätzung zu den Gesamtkosten des Therapieablaufs getroffen werden, wenn sich die Kenngröße auf die Behandlungskosten bezieht. Auch diese können die Kenngröße oder einen Indikator darstellen.

Die Kenngröße bzw. Simulation kann auch mehrere weitere Therapiestationen bzw. Ketten mehrerer weitere Therapiestationen betreffen, so dass eine Erfolgswahrscheinlichkeit bzw. Ressourcen, Aufwand etc. über einen Therapieablauf bis zur vollständigen oder maximal möglichen Wiederherstellung prognostiziert, angezeigt ausgeben und/oder einer Auswahl bzw. Freigabe der weiteren Therapiestation zugrunde gelegt werden können.

Die Kenngröße(n) kann/können vorzugsweise, insbesondere zusätzlich und/oder zugeordnet zu der oder den weiteren Therapiestationen bzw. hierzu korrespondierender Parameter, über die Benutzerschnittstelle ausgegeben werden.

### Ranking

Vorzugsweise erfolgt ein automatisiertes Ranking der weiteren Therapiestation(en) unter Berücksichtigung eines Grads der Eignung und/oder der Verfügbarkeit von Ressourcen für die Therapiestation und/oder hierzu korrespondierender Kenngrößen. Das Ranking, Kenngrößen und/oder Therapiestationen anhand des Rankings können vorzugsweise durch die Benutzerschnittstelle ausgegeben werden.

So kann durch das vorschlagsgemäße Verfahren ermittelt werden, ob die Therapieprognosen bzw. Kenngröße durch Warten auf eine geeignetere Therapiestation oder durch Wahl einer weniger geeigneten, dafür früher verfügbaren Therapiestation besser ist. Entsprechend kann das Ranking diejenige Therapiestation priorisieren, die die beste Therapieprognose bzw. Kenngröße ergibt. Alternativ oder zusätzlich kann/können für das Ranking weitere, die jeweilige Therapiestation charakterisierende Größen berücksichtigt werden, beispielsweise Nebenwirkungen, Aufwand und/oder Kosten.

In einem weiteren Aspekt werden nicht nur eine oder mehrere potenziell unmittelbar folgende Therapiestation, sondern zusätzlich weitere, wahrscheinlich nach Abschluss der ermittelten Therapiestation angezeigte erforderliche weitere Therapiestationen ermittelt. Auf diese Weise können Therapiestation-Folgen bestimmt werden. Das zuvor beschriebene Vorgehen bzw. Ranking kann diese Therapiestation-Folgen und die damit einhergehenden benötigten Ressourcen sowie sonstige die Therapiestationen der Therapiestations-Folgen charakterisierende Größen oder Kenngrößen berücksichtigen.

Das Ranking kann für eine Vorauswahl von Therapiestationen genutzt werden oder eine Vorauswahl von Therapiestationen kann in Zusammenhang mit der Erstellung des Rankings erfolgen. Auf diese Weise ist es möglich, die potenzielle Auswahl folgender Therapiestationen auf besonders geeignete Therapiestationen zu fokussieren oder zu beschränken.

Eine Arbeitsanweisung bzw. Handlungsempfehlung kann das ermittelte Ranking und/oder die ermittelte Auswahl von Therapiestationen sein oder repräsentieren oder hierzu korrespondieren. Es ist hierbei bevorzugt, dass das Ranking und/oder die Vorauswahl, beispielsweise als Arbeitsanweisung bzw. Handlungsempfehlung oder Teil hiervon, durch die Benutzerschnittstelle ausgegeben bzw. zur Auswahl angeboten werden. Auf diese Weise kann der Benutzer zwischen möglichen, sinnvollen und verfügbaren Therapiestationen wählen.

### Ausgabe

Die Ausgabe ist oder umfasst vorzugsweise eine oder mehrere weitere Therapiestationen bzw. hierzu korrespondierende Parameter oder Datensätze (ganz oder teilweise). Alternativ oder zusätzlich korrespondiert die Ausgabe hierzu oder ist hierzu zugeordnet oder repräsentiert die eine oder mehreren weiteren Therapiestationen, hierzu korrespondierende Parameter oder Datensätze.

Die Ausgabe kann in Form einer Arbeitsanweisung und/oder Handlungsempfehlung an einen Benutzer erfolgen. Die Arbeitsanweisung und/oder Handlungsempfehlung korrespondiert vorzugsweise zu der oder den weiteren, insbesondere dritten, Therapiestation(en). Dabei umfasst die Arbeitsanweisung bzw. Handlungsempfehlung bevorzugt eine oder mehrere Therapieschritte im Zusammenhang mit der weiteren bzw. dritten Therapiestation und/oder betrifft eine therapeutische Maßnahme, insbesondere Medikation, medizinische Untersuchung und/oder Rehabilitationsmaßnahme.

Besonders bevorzugt korrespondiert die Arbeitsanweisung und/oder Handlungsempfehlung zu einem Ablauf einer bestimmten Therapiestation. Als Arbeitsanweisung und/oder Handlungsempfehlung können therapiestationsspezifische Orte, Zeiten, Abfolgen technischer Maßnahmen und hierbei zugrundezulegende Parameter ausgegeben werden, insbesondere chronologisch und/oder als Reihe, Liste, Tabelle, Aufstellung o.dgl.

Die Arbeitsanweisung bzw. Handlungsempfehlung umfasst vorzugsweise Informationen, die für einen reibungslosen Übergang von einer Therapiestation zu einer anderen Therapiestation, insbesondere von einer Phase zur nachfolgenden Phase einer Therapie, notwendig oder sinnvoll sind. Anders als bei bekannten Ansätzen zur informationstechnischen Unterstützung bei einem Therapieablauf werden vorzugsweise nicht nur die wesentlichen Parameter der aktuellen Therapiestation herangezogen, sondern die Parameter von unterschiedlichen, vorzugsweise bereits erfolgten, Therapiestationen dazu genutzt, die weitere Therapiestation zu ermitteln und/oder den Übergang zu der weiteren Therapiestation zu verbessern, also möglichst optimal zu gestalten. Hierbei können Besonderheiten im individuellen Therapieablauf eines konkreten Patienten berücksichtigt werden.

Während eine Handlungsempfehlung insbesondere an einen menschlichen Benutzer gerichtet ist, umfasst der Begriff der Arbeitsanweisung im Sinne der Erfindung allgemein jede insbesondere mehrschrittige Anweisung zur Art und Weise der Durchführung eines Arbeitsablaufs, beispielsweise mit oder für ein Medizingerät.

Ein solcher Arbeitsablauf ist vorzugsweise Teil einer Therapiestation im Therapieablauf für einen Patienten oder repräsentiert die Therapiestation. Durch die eine Arbeitsanweisung und/oder Handlungsempfehlung umfassende Ausgabe durch die Benutzerschnittstelle wird also eine Therapiestation vorzugsweise zumindest teilweise charakterisiert.

Vorzugsweise werden zur informationstechnischen Unterstützung durch oder als Ausgabe bzw. durch oder mittels der Benutzerschnittstelle Informationen zur Verfügung gestellt. Die Informationen betreffen vorzugsweise einen folgenden Schritt innerhalb des Therapieablaufs oder geben diesen oder Optionen hierfür an, insbesondere als, in Form von und/oder korrespondierend zu einer oder mehreren weiteren Therapiestationen.

Die Arbeitsanweisung und/oder Handlungsempfehlung für den Benutzer erleichtert zum einen die durchzuführenden Arbeitsabläufe, da in einem Beispiel eine gegebene Arbeitsanweisung schlicht befolgt wird. Alternativ oder zusätzlich wird die Fehleranfälligkeit gesenkt, insbesondere wenn, wie bevorzugt, die Arbeitsanweisung bzw. Handlungsempfehlung alle erforderlichen und durchzuführenden Maßnahmen umfasst, die vom Benutzer an der betreffenden Therapiestation umzusetzen sind, so dass ein Übersehen oder Vergessen oder eine terminliche Überschneidung oder Kollision von Maßnahmen unwahrscheinlich ist.

Die Arbeitsanweisung bzw. Handlungsempfehlung kann insbesondere eine durchzuführende Therapie oder medizinische Untersuchung und/oder Besonderheiten bei einer solchen Therapie oder Untersuchung betreffen. Beispielsweise können Daten bezüglich einer möglichen, empfohlenen, verordneten und/oder anzuwendenden Medikation für einen Patienten durch die Benutzerschnittstelle ausgegeben werden. Die Arbeitsanweisung bzw. Handlungsempfehlung kann eine medizinische Untersuchung oder Behandlung oder auch eine Rehabilitationsmaßnahme betreffen.

Gemäß einem weiteren, auch unabhängig realisierbaren Aspekt der vorliegenden Erfindung kann die bzw. eine von mehreren Benutzerschnittstellen eine Anzeigeeinrichtung mit einer organisatorischen, insbesondere chronologischen, Auflistung von Parametern korrespondierend zu derselben Therapieeinrichtung bzw. Ressource in Hinblick auf die Therapiestationen unterschiedlicher Patienten sein oder aufweisen.

Die Anzeigeeinrichtung kann im Stil einer "Boardingtafel" ausgeführt sein, wie sie in vergleichbarer Weise aus Flughäfen oder Bahnhöfen zur Anzeige von Starts, Landungen, Ankünften und/oder Abfahrten bekannt sind. Die angezeigten oder anzeigbaren Einträge können das Ergebnis der Verarbeitung der Parameter bzw. Datensätze sein. Insbesondere werden bei öffentlich zugänglichen Anzeigeeinrichtungen jedoch nur organisationsrelevante Parameter ausgegeben. Bei Anzeigeeinrichtungen in nicht öffentlich zugänglichen Bereichen können alternativ oder zusätzlich auch therapierelevante Parameter ausgegeben werden.

### Freigabe

Vorzugsweise weist die bzw. eine von mehreren möglichen Ausgaben durch die Benutzerschnittstelle eine Mehrzahl von Therapievorschlägen bzw. weiteren Therapiestationen als Optionen auf oder ist hierdurch gebildet. Diese Mehrzahl von Therapievorschlägen kann durch den Parametervergleich bzw. wie zuvor beschrieben durch gemeinsame Verarbeitung von Parametern unterschiedlicher Therapiestationen ermittelt werden.

Vorzugsweise werden also mehrere mögliche und zueinander alternative weitere Therapiestationen oder Folgen von Therapiestationen ermittelt. Diese können dann als Optionen zur Auswahl bzw. Freigabe ausgegeben werden.

Eine oder mehrere dieser Optionen können, insbesondere nach einer Freigabe, vorzugsweise durch einen Arzt, über die Benutzerschnittstelle in Form einer Arbeitsanweisung ausgegeben werden, die beispielsweise vom Pflegepersonal zur Durchführung von entsprechenden Therapiemaßnahmen umgesetzt werden soll.

Der Auswahlprozess einer nächsten Therapiestation kann zwei- oder mehrschrittig erfolgen. Hierzu ist bevorzugt, dass in einem ersten Schritt die Freigabe einer Option erfolgt und in einen weiteren Schritt eine Auswahl zwischen den und/oder eine, insbesondere terminliche und/oder organisatorische, Konkretisierung der freigegebenen Optionen erfolgt. Die Schritte können mit dem selben oder unterschiedlichen Benutzerschnittstellen bzw. Teilen der Benutzerschnittstelle, insbesondere unterschiedlichen Anzeigemitteln erfolgen.

Insbesondere ist vorgesehen, dass zunächst eine erste Vorauswahl bzw. ein Ranking von Optionen durch die Benutzerschnittstelle zur Freigabe ausgegeben und mittels der Benutzerschnittstelle ein oder mehre der Therapiestationen freigebbar und/oder modifizierbar bzw. konkretisierbar sind, beispielsweise durch einen Arzt. Auf diese Weise kann eine oder können mehrere mögliche weitere Therapiestationen zunächst automatisch ermittelt und dann durch die Freigabe selektiert werden.

Besonders bevorzugt werden bei oder durch die Ermittlung der Optionen oder hierzu korrespondierender Kenngrößen die für diese Therapiestation benötigten Ressourcen automatisch bzw. systemseitig ermittelt, berücksichtigt und/oder (vor)reserviert. Hierzu kann ein Ressourcenmangementsystem vorgesehen sein, beispielsweise eine Schnittstelle zu einem Krankenhausinformationssystem, in dem Belegzeiten für Geräte, Räume, Personal o.dgl. gehandhabt wird. Alternativ oder zusätzlich werden durch eine Datenbank Belegzeiten für Geräte, Räume, Personal oder sonstige Ressourcen als für den für die ausgewählte Therapiestation benötigte Dauer als belegt gekennzeichnet. Hierdurch kann eine Doppelbelegung von Ressourcen verhindert werden und die Auslastung verbessert werden.

Aus Sicherheitsgründen ist vorzugsweise vorgesehen, dass die Optionen zunächst freigeben werden müssen, bevor sie in einem weiteren Schritt ausgewählt werden können. Insbesondere ist also vorgesehen, dass die dritte oder weitere Therapiestation bzw. Arbeitsanweisung und/oder die Handlungsempfehlung, insbesondere in Form verschiedener Therapiemöglichkeiten im Zusammenhang mit einer weiteren Therapiestation, vor ihrer Umsetzung, unabhängig davon, ob diese automatisch oder manuell erfolgt, zunächst freigegeben werden muss.

Die Freigabe erfolgt dabei insbesondere mittels eines Freigabesignals nach einer entsprechenden Freigabe durch einen Benutzer, beispielsweise durch den verantwortlichen Arzt, über die oder eine weitere Benutzerschnittstelle. Hierdurch wird eine zusätzliche Kontrollinstanz bzw. -möglichkeit geschaffen, durch die vermieden wird, dass Fehler im Ablauf des Verfahrens, beispielsweise durch Hardware- und/oder Softwarefehler, negative Auswirkungen auf den Therapieablauf eines Patienten haben.

Das Freigabesignal kann insbesondere eine Auswahl einer oder mehrerer der Optionen bzw. von verschiedenen Therapiemöglichkeiten oder Therapiestationen umfassen, die mit der Gesamtheit von Therapiemöglichkeiten bzgl. weiteren Therapiestationen in Verbindung gesetzt wird.

Die Freigabe kann beispielsweise durch eine Eingabe über die Benutzerschnittstelle erfolgen. Insbesondere ist vorgesehen, dass durch eine Auswahl von einzelnen Optionen in einer ausgegebenen Liste von Optionen die Freigabe erfolgt. Den Optionen können jeweils ein oder mehrere Freigabeparameter, beispielsweise ein Flag oder eine sonstige Markierung, zugeordnet sein, der den Freigabestatus repräsentiert. Dies ermöglich es im Folgenden nicht freigegebene Optionen zu verwerfen oder nicht zur Auswahl anzubieten.

Die jeweilige Option und/oder der Freigabeparameter kann/können in einem Datensatz oder mehreren Datensätzen gespeichert werden. Hierbei kann ein neuer, den oder die Freigabeparameter aufweisender Datensatz angelegt werden oder der oder die Freigabeparameter können zusammen mit den Optionen in einem gemeinsamen Datensatz gespeichert werden. Hier sind aber auch weitere Lösungen denkbar.

In einem nachfolgenden Schritt können von einem Freigabemittel entsprechend der Auswahl bzw. entsprechend den Freigabeparametern nur bestimmte Arbeitsanweisungen bzw. Handlungsempfehlungen zur Ausgabe durch die Benutzerschnittstelle und/oder zur Weiterverarbeitung freigegeben werden. Die nicht freigegebenen Optionen können verworfen werden oder alternativ mit den freigegebenen Optionen zusammen weiterübermittelt werden, wobei sie aufgrund der Zuordnung der Freigabeparameter bei der Weiterverarbeitung und/oder Ausgabe durch die Benutzerschnittstelle unberücksichtigt bleiben können.

In einem zweiten Schritt kann über dieselbe oder eine andere Benutzerschnittstelle, beispielsweise durch den Patienten, eine Hilfsperson des Patienten, medizinisches oder verwaltungstechnisches Personal o.dgl., eine konkrete der freigegebenen Optionen ausgewählt und/oder organisatorisch weiter konkretisiert werden. Auf diese Weise ist beispielsweise eine automatische Terminvergabe möglich.

Vorzugsweise wird durch die Auswahl der konkreten bzw. zuvor freigegebenen Option die hierfür benötigten Ressourcen automatisch reserviert, beispielsweise über ein Ressourcenmanagementsystem bzw. Ressourcenkalender etc. Insbesondere werden vorreservierte Ressourcen automatisch fest reserviert, wenn die hierzu korrespondierende Option ausgewählt wird. Ressourcen korrespondierend zu nicht ausgewählten Optionen können dann freigegeben werden. Alternativ zu einer Vorreservierung kann auch eine Verfügbarkeitsprüfung durch Auswahl der korrespondierenden Option ausgelöst werden und die Auswahl nur im Fall der Verfügbarkeit von Ressourcen erfolgen.

**Parameterermittlung**Ein oder mehrere der Parameter, insbesondere Ergebnisparameter, können (automatisch) mittels Sensoren erfasst werden. Hierbei handelt es sich insbesondere um Sensoren zur Messung von Vitalparametern, wie beispielsweise Blutdruck, Herzfrequenz oder neuronaler Aktivität.

Alternativ oder zusätzlich können ein oder mehrere der Parameter, insbesondere Ergebnisparameter, auch registriert werden, indem manuell über die Benutzerschnittstelle eingegebene Daten oder Werte als Parameter in Form von Datensätzen in die Datenbank eingespeist und dort gespeichert werden.

Bevorzugt ist zudem das Auslesen bzw. Einspeisen von einem oder mehreren Parametern, insbesondere Ergebnisparametern, von einem medizinisch-diagnostischen Gerät und/oder von einem Ressourcenmanagementsystem, wie es beispielsweise prinzipiell aus dem Logistikbereich bekannt ist.

Vorzugsweise werden die Parameter, insbesondere Ergebnisparameter, zentral, insbesondere in einer zentralen Datenbank und/oder als Datensatz und/oder in einem Datensatz, insbesondere als oder in dem zu der jeweiligen Therapiestation korrespondierenden Datensatz gespeichert. Dazu kann insbesondere an einem Standort einer medizinisch-therapeutischen Einrichtung ein zentrales Rechenzentrum oder eine informationstechnologische Einrichtung, beispielweise ein Server, vorgesehen sein, welche die zentrale Datenbank beinhaltet.

### System

Zur Durchführung des zuvor beschriebenen Verfahrens ist nach einem weiteren, auch unabhängig realisierbaren Aspekt der vorliegenden Erfindung ein System zur Unterstützung bei einem Therapieablauf für einen Patienten vorgesehen.

Das System weist vorzugsweise wenigstens eine Datenbank zur Speicherung von Datensätzen, wenigstens eine, insbesondere zentrale, Datenverarbeitungseinrichtung zur Verarbeitung von Datensätzen und wenigstens eine Benutzerschnittstelle zur Eingabe und/oder Ausgabe von Datensätzen auf.

Eine Ausgabe erfolgt durch die Benutzerschnittstelle auf Basis mindestens zweier, zu unterschiedlichen Therapiestationen, insbesondere zu einer ersten Therapiestation und einer zweiten Therapiestation, korrespondierender, patientenspezifische und/oder therapierelevante Parameter aufweisender Datensätze.

Hierbei ist bevorzugt, dass die Ausgabe zu einer weiteren Therapiestation korrespondiert und/oder eine, insbesondere die Medikation, eine medizinische Untersuchung und/oder eine Rehabilitationsmaßnahme betreffende Arbeitsanweisung und/oder Handlungsempfehlung an einen Benutzer umfasst.

Die Datenverarbeitungseinrichtung dient insbesondere der Durchführung von insbesondere logischen und/oder kalkulatorischen Operationen mit Datensätzen, die in der Datenbank gespeichert sind. Dazu werden die Datensätze aus der Datenbank ausgelesen und vorzugsweise algorithmisch von der Datenverarbeitungseinrichtung miteinander verknüpft.

Mögliche Algorithmen, die auf die Datensätze angewendet werden können, sind beispielsweise Sortier- und/oder Rechenoperationen oder ein Vergleich der Datensätze oder Parameter dieser miteinander und/oder mit vorgegebenen Referenzdaten. Darüber hinaus versteht es sich, dass die aus der Datenbank ausgelesenen Datensätze auch unverändert über die Benutzerschnittstelle ausgegeben werden können oder an eine weitere Datenverarbeitungseinrichtung weitergeleitet werden können.

### Schnittstellen

Gemäß einem weiteren Aspekte der vorliegenden Erfindung sind mehrere Therapiestationen in einer Phase zusammengefasst und zwischen zwei Phasen ist eine Schnittstelle vorgesehen. Die Schnittstelle ist vorzugsweise dazu ausgebildet, den Übergang zwischen zwei Phasen zu steuern und zu optimieren.

Insbesondere korrespondieren unterschiedliche Phasen jeweils zu unterschiedlichen Einrichtungen, insbesondere eine Phase zu einer Klinik und eine folgende Phase zu einer Rehabilitationseinrichtung. Insbesondere sind folgende oder ein Teil der folgenden Phasen vorgesehen:
- Phase I:: Ambulant ärztlicher Bereich (Kassenarzt)
- Phase II:: Akutbereich
- Phase III:: Aktivierungspflege
- Phase IV:: stationäre Rehabilitation
- Phase V:: ambulante Rehabilitation
- Phase VI:: Nachsorge

Die Schnittstellen können dazu ausgebildet, sein, den Übergang in eine folgende Phase durch Anpassung oder Definition von Therapiestationen der aktuellen und/oder der folgenden Phase zu steuern.

Ferner kann durch die Schnittstellen eine Freigabe zum Wechsel von einer in die nächste Phase blockiert oder freigegeben werden.

Insbesondere ist vorgesehen, dass die gemeinsame Verarbeitung von zu unterschiedlichen, bereits erfolgten Therapiestationen zu einer Definition, einem Vergelich und/oder einer Bewertung von Bedingungen wie Schwellwerten o.dgl. genutzt wird, so dass die Schnittstelle, vorzugsweise bei Erreichen der Bedingungen bzw. Schwellwerte, automatisch einen möglichst reibungslosen Übergang in die Folgephase und/oder Vorbereitungen hierzu einleitet.

Zu den Vorbereitungen können automatische Ressourcenermittlungen für Therapiestationen der folgenden Phase, Ressourcenreservierungen für Therapiestationen der folgenden Phase und/oder Informationsübermittlungen an die aktuelle oder die Einrichtung der folgenden Phase gehören.

### Benutzerschnittstellen

Es können auch mehrere Benutzerschnittstellen vorgesehen sein oder die Benutzerschnittstelle kann zu unterschiedlichen Zeitpunkten oder an unterschiedlichen Orten durch unterschiedliche Benutzer Verwendung finden oder durch unterschiedliche Hardware gebildet sein. So kann durch die Benutzerschnittstelle eine Empfehlung für eine nächste Therapiestation bzw. Optionen ausgegeben werden, die durch den Benutzer, beispielsweise einen Arzt, zunächst durch Abänderung und/oder Bestätigung freigegeben bzw. verordnet wird. Die Empfehlung wird vorzugsweise durch die Parameter bzw. Datensätze ermittelt. Optional kann eine Auswahl und/oder (organisatorische bzw. terminliche) Konkretisierung durch den Benutzer, insbesondere einen anderen Benutzer bzw. Patienten und/oder eine andere Benutzerschnittstelle, erfolgen.

Daraufhin kann die oder eine andere Benutzerschnittstelle die Durchführung der Therapiestation führen oder begleiten. Beispielsweise erinnert die Benutzerschnittstelle im Vorfeld an eine anstehende Therapiestation und führt durch diese, indem die Therapiestation charakterisierende Informationen ausgegeben werden. Das vorschlagsgemäße System ist also bevorzugt zur Benutzerführung eingerichtet.

Bei einer bevorzugten Ausführungsform weist die Benutzerschnittstelle eine insbesondere elektronische Schautafel auf oder ist hierdurch gebildet. Durch eine entsprechende Platzierung der Schautafel, beispielsweise in einem öffentlichen Bereich, ist es einer größeren Anzahl von Benutzern gleichzeitig möglich, die vom System ausgegebenen Informationen aufzunehmen. Mittels der Schautafel können beispielsweise Informationen bzgl. der bisherigen und/oder verbleibenden Verweildauer eines Patienten an einer Therapiestation, Kenngrößen wie den Patientenstatus, insbesondere bzgl. seiner Mobilität und/oder zutreffender ICD bzw. ICF Klassen, sowie Informationen zur nächsten Therapiestation, in Frage kommende Therapiemöglichkeiten/Therapiestationen und/oder ein ökonomischer Indikator als Kenngröße, insbesondere in Zusammenhang mit dem Verlauf oder weiteren Therapiestationen ausgegeben werden.

Alternativ oder zusätzlich kann die Benutzerschnittstelle ein Mittel zur akustischen Signalisierung aufweisen. Hierdurch wird die Signalwirkung im Zusammenhang mit über die Benutzerschnittstelle wiedergegebenen Informationen unterstützt. Die Aufmerksamkeit des oder der Benutzer wird dadurch auf die Benutzerschnittstelle und die dort wiedergegebenen Informationen gerichtet. Dies kann beispielsweise von Vorteil sein, wenn über die Benutzerschnittstelle aus medizinischer Sicht kritische, oder aus diesen oder sonstigen Gründen als besonders relevant und/oder eilbedürftige Informationen wiedergegeben werden, wie es beispielsweise in Notfallsituationen der Fall ist. Solche Informationen werden vorzugsweise automatisch erkannt und die Signalisierung ausgelöst.

Da die Benutzerschnittstelle neben der Ausgabe vorzugsweise auch zur Eingabe von Parametern in Form von Datensätzen dient, kann durch ein Mittel zur akustischen Signalisierung auch ein Bestätigungssignal abgegeben werden, wenn eine Eingabe erfolgreich abgeschlossen und/oder an die Datenbank übermittelt wurde.

Vorzugsweise ist die Benutzerschnittstelle zur insbesondere standortunabhängigen Eingabe und/oder Ausgabe von Datensätzen ausgestaltet. Anstelle einer Zuordnung der Benutzerschnittstelle zu einer festen Einrichtung, wie beispielsweise einem fest installierten Computerterminal, ist die Benutzerschnittstelle dabei bevorzugt als Software für eine tragbare Kommunikationseinrichtung, d. h. als sogenannte App, beispielsweise für ein Smartphone und/oder einen Tablet-Computer, ausgestaltet, so dass ein Benutzer die Ein- bzw. Ausgabe von Datensätzen über die Benutzerschnittstelle in Form eines von ihm mitgeführten tragbaren Geräts grundsätzlich an jedem Ort möglich ist, an dem eine Verbindung zum erfindungsgemäßen System besteht.

Durch den standortunabhängigen Zugriff auf die Datensätze im System stehen benötigte Informationen schneller zur Verfügung und insbesondere organisatorische Abläufe werden erheblich beschleunigt. Dies kann im medizinischen Umfeld, insbesondere im Bereich der Intensivbetreuung und/oder Notfallmedizin, von Bedeutung sein.

Ferner kann die Benutzerschnittstelle auch als Webseite oder Online-Portal ausgestaltet sein. Dies ermöglicht den Zugriff, d. h. den Abruf und die Eingabe von Datensätzen bzw. Parametern dieser, von nahezu jedem Ort mit einer Internet- bzw. Netzwerkanbindung. Auf diese Weise können beispielsweise Patienten Daten über ihren Gesundheitszustand oder ihr Befinden selbstständig, insbesondere von ihrem Wohnort aus, eingeben. Des Weiteren ermöglicht ein solcher Online-Zugang zum erfindungsgemäßen System niedergelassenen Ärzten, den Therapieablauf ihres Patienten betreffende Daten mit geringem Aufwand von nahezu beliebiger Stelle aus einzusehen und/oder zu ergänzen bzw. zu beeinflussen. Vorzugsweise setzen Abrufe bzw. Zugriffe eine Verschlüsselung bzw. Verifikation voraus bzw. umfassen diese.

### Positionsbestimmung

Bei einer bevorzugten Ausgestaltung des Systems ist wenigstens ein Mittel zur Registrierung und/oder zur Positionsbestimmung von Gegenständen und/oder Personen vorgesehen. Auf diese Weise lassen sich Ressourcen, wie beispielsweise Lagerbestände von Verbrauchsmaterial, leicht erfassen und durch Übermittlung eines entsprechenden Registrierungssignals an die Datenverarbeitungseinrichtung und/oder an die Datenbank weiterverarbeiten.

Alternativ oder zusätzlich lassen sich bestimmte Gegenstände, wie beispielsweise eine physische Patientenakte oder ein tragbares medizinisches Gerät, und/oder Personen, wie beispielsweise Ärzte oder Teile des Pflegepersonals, innerhalb einer medizinisch-therapeutischen Einrichtung lokalisieren.

Es werden vorzugsweise Datensätze mit entsprechenden Ortungsinformationen erzeugt und in der Datenbank gespeichert bzw. mittels der Datenverarbeitungseinrichtung verarbeitet. Hierdurch lässt sich der Einsatz von Personal und/oder materiellen Ressourcen planen und optimieren.

Bevorzugt werden Statusinformationen zusätzlich oder alternativ zu den Positionsangaben übertragen. Dies betrifft insbesondere die Verfügbarkeit von Geräten, Personal sowie darüber hinaus insbesondere auch von Räumlichkeiten.

### Computerprogrammprodukt

Die vorliegende Erfindung betrifft in einem auch unabhängig realisierbaren Aspekt ferner ein Computerprogrammprodukt, insbesondere ein computerlesbares Speichermittel, Speichermedium oder ein Computer, Microcontroller oder sonstiges Gerät mit einem Prozessor.

Das Computerprogrammprodukt weist Programmcodemittel auf, die dazu ausgebildet sind, das vorschlagsgemäße Verfahren bzw. die Verfahrensschritte des vorschlagsgemäßen Verfahrens auszuführen oder zu bewirken.

Die Programmcodemittel werden vorzugsweise auf einem Computer oder Prozessor ausgeführt, wodurch das Verfahren bzw. die Verfahrensschritte ausgeführt werden.

Insbesondere kann es sich bei dem Computerprogrammprodukt um einen Datenträger, eine Netzwerkressource wie eine Datenbank, ein FTP-Server o.dgl. mit einem Programm zur Ausführung des Verfahrens. Alternativ oder zusätzlich handelt es sich um ein rechner- oder computerbasiertes Gerät oder ein Verbund, Netzwerk, und/oder System mit einem oder mehreren rechner- oder computerbasierten Geräten mit den Programmcodemitteln zur Durchführung des Verfahrens.

### Eigenschaften und Vorteile

In besonders vorteilhafter Weise lässt sich das vorschlagsgemäße Verfahren und/oder das vorschlagsgemäße System im Bereich der Akutversorgung, der Pflege und/oder der Rehabilitationsbehandlung von Patienten einsetzen. Bevorzugt wird das Verfahren und/oder das System therapiebegleitend in allen der vorgenannten Bereiche verwendet bzw. in medizinisch-therapeutischen Einrichtungen, die den entsprechenden Bereichen zugeordnet sind, eingesetzt.

Bei dem Verfahren wird ein Therapieablauf vorzugsweise informationstechnisch unterstützt. Grundsätzlich betrifft das Verfahren Verbesserungen oder Optimierungen eines Therapieablaufs eines Patienten mit technischen, besonders bevorzugt informationstechnischen, Mitteln wie vernetzten Datenverarbeitungseinrichtungen, Datenbanken, Anzeigeeinrichtungen und dergleichen. Die informationstechnische Unterstützung liegt besonders bevorzugt in einer unmittelbaren Führung oder Anleitung eines Patienten. Alternativ oder zusätzlich wird eine Hilfsperson oder eine Hilfseinrichtung des Patienten, beispielsweise ein Arzt, Pfleger, Assistent, oder elektronischer Assistent, geführt oder mit Informationen zur Führung des Patienten versorgt.

Gegenüber dem Einsatz bekannter elektronischer, informationstechnologischer Unterstützungssysteme in den einzelnen Einrichtungen ergibt sich durch das erfindungsgemäße Verfahren und/oder System der Vorteil, dass Abläufe und Übergänge an den Schnittstellen zwischen den verschiedenen Einrichtungen bzw. zwischen Phasen oder allgemein zwischen verschiedenen Therapiestationen eines Therapieablaufs für einen Patienten reibungsloser, d. h. insbesondere ohne längere Unterbrechungszeiten und ohne vermeidbare Doppeluntersuchungen, erfolgen. Hieraus ergibt sich neben einem offensichtlichen Kostenvorteil häufig auch ein verbesserter Therapieerfolg, da die einzelnen Behandlungen und Nachbehandlungen nahtlos aneinander anschließen, unter den verfügbaren Optionen therapieoptimal ermittelt und ausgewählt und/oder im kleinsten aus medizinischer Sicht sinnvollen zeitlichen Abstand aufeinanderfolgen können.

Weitere Merkmale, Vorteile, Eigenschaften und Anwendungsmöglichkeiten der vorliegenden Erfindung ergeben sich aus den Ansprüchen und der nachfolgenden Beschreibung von Ausführungsbeispielen anhand der Zeichnung und der Zeichnung selbst. Dabei bilden alle beschriebenen und/oder bildlich dargestellten Merkmale für sich oder in beliebiger Kombination den Gegenstand der vorliegenden Erfindung, unabhängig von ihrer Zusammenfassung in den Ansprüchen oder deren Rückbeziehung.

Es zeigt
- Fig. 1: eine schematische Darstellung der Grundstruktur des erfindungsgemäßen Verfahrens;
- Fig. 2: eine schematische Darstellung eines besonders bevorzugten Aspekts des erfindungsgemäßen Verfahrens;
- Fig. 3: eine schematische Darstellung eines durch das erfindungsgemäße Verfahren informationstechnisch unterstützten Therapieablaufs für einen Patienten;
- Fig. 4: eine schematische Darstellung eines Teilabschnitts eines durch das erfindungsgemäße Verfahren informationstechnisch unterstützten Therapieablaufs für einen Patienten;
- Fig. 5: eine schematische Darstellung eines weiteren Aspekts des erfindungsgemäßen Verfahrens;
- Fig. 6: eine schematische Darstellung einer bevorzugten Ausführungsform des erfindungsgemäßen Systems; und
- Fig. 7: eine schematische Darstellung vorschlagsgemäßer Datensätze.

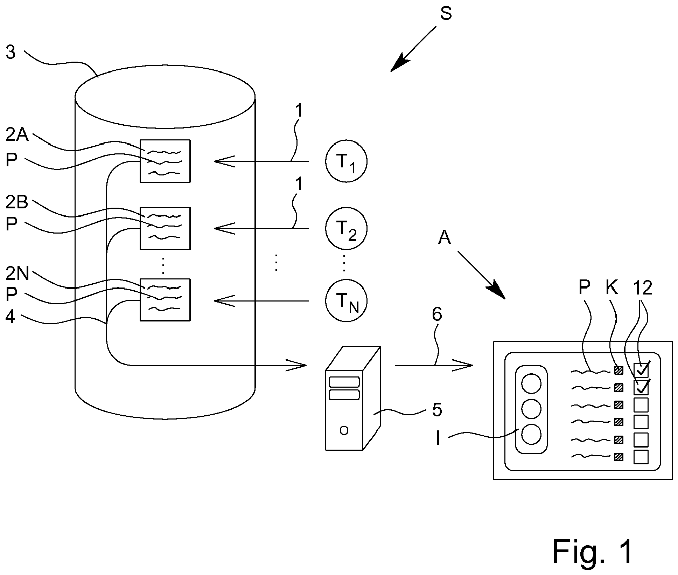

In Fig. 1 ist ein Ablauf des vorschlagsgemäßen Verfahrens schematisch wiedergegeben.

Zu einer ersten Therapiestation T₁ korrespondiert vorzugsweise ein erster Datensatz 2A. Vorzugsweise sind Parameter P vorgesehen, die zu der ersten Therapiestation T₁ korrespondieren und den ersten Datensatz 2A oder einen Teil hiervon bilden.

Zu einer zweiten Therapiestation T₂ korrespondiert vorzugsweise ein zweiter Datensatz 2B. Vorzugsweise sind Parameter P vorgesehen, die zu der zweiten Therapiestation T₂ korrespondieren und den zweiten Datensatz 2B oder einen Teil hiervon bilden.

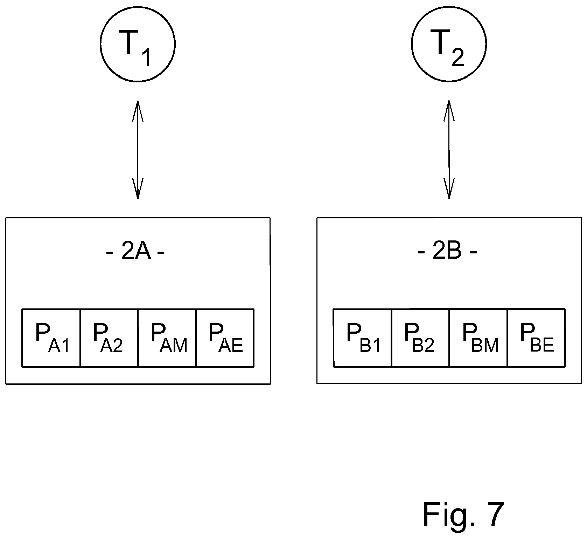

Anhand der Darstellung in Fig. 7 kann der innere, strukturelle Aufbau zweier Datensätze 2, nämlich des ersten Datensatzes 2A und des zweiten Datensatzes 2B, verdeutlicht werden. Die Datensätze 2A, 2B, die mit der ersten Therapiestation T₁ bzw. der zweiten Therapiestation T₂ korrespondieren, weisen jeweils eine Reihe von Parametern P auf, durch welche die jeweils korrespondierende Therapiestation T₁, T₂ charakterisiert wird.

Hierbei kann ein organisatorischer Parameter P_{A1} des ersten Datensatzes 2A beispielsweise einen zur ersten Therapiestation T₁ korrespondierenden Ort betreffen.

Ein weiterer organisatorischer Parameter P_{A2} betrifft vorzugsweise einen der ersten Therapiestation T₁ zugeordneten Zeitpunkt.

In entsprechender Weise kann der zweite Datensatz 2B Parameter P_{B1}, P_{B2} aufweisen, die sich ihrerseits auf den der zweiten Therapiestation T₂ zugeordneten Ort bzw. Zeitpunkt beziehen.

Der mit einer Therapiestation T₁, T₂, T₃, T_{N} korrespondierende Zeitpunkt kann grundsätzlich in der Zukunft liegen, auch wenn er für die erste und zweite Therapiestation T₁, T₂ bevorzugt in der Vergangenheit liegt.

Ferner können die Datensätze 2A, 2B jeweils wenigstens einen therapierelevanten Parameter P_{AM}, P_{BM} aufweisen, der/die eine mit der zugehörigen Therapiestation T₁, T₂ in Zusammenhang stehende Maßnahme betrifft. Diese Parameter P_{AM}, P_{BM} korrespondieren bevorzugt zu einer durchzuführenden Untersuchung oder medizinischen Behandlung oder auch einem Patienten zu verabreichende Medikamente, während weitere in den Datensätzen 2A, 2B enthaltenen Parameter P_{A2}, P_{B2} beispielsweise den Verabreichungszeitpunkt angeben können.

Die Datensätze 2A, 2B weisen darüber hinaus, vorzugsweise, auch Ergebnisparameter P_{AE}, P_{BE} auf, die insbesondere mit einem bei einer vergangenen Therapiestation T₁, T₂ erreichten und/oder bei einer zukünftigen Therapiestation T₁, T₂ zu erreichenden Ergebnis, beispielsweise einem Therapieerfolg, in Zusammenhang stehen, zu dem Ergebnis korrespondieren oder dieses repräsentieren. Die Ergebnisparameter P_{AE}, P_{BE} können therapierelevante, insbesondere diagnostische und/oder therapeutische, Parameter P_{AE}, P_{BE} sein.

Zu einer weiteren Therapiestationen T_{N} korrespondiert vorzugsweise ein weiterer Datensatz 2N.

Es können auch zwei oder mehr weitere Therapiestationen T_{N} mit jeweils korrespondierenden Datensätzen 2N vorgesehen sein.

Parameter P_{N1}, P_{N2}, P_{NM}, P_{NE} des/der weiteren Datensatzes / Datensätze 2N können den zuvor in Zusammenhang mit dem ersten und/oder zweiten Datensatz 2A, 2B erläuterten Parametern P entsprechen.

Die Datensätze 2A, 2B, 2N bzw. deren Parameter P sind vorzugsweise in einer Datenbank 3 abgelegt bzw. abgespeichert.

Vorzugsweise korrespondieren der erste, zweite und/oder weitere Datensatz 2A, 2B, 2N zu bereits abgeschlossenen Therapiestationen T₁, T₂, T_{N}.

Parameter P korrespondierend zu der ersten Therapiestation T₁ können über einen Eingabekanal 1, vorzugsweise als Datensatz 2A oder Teil hiervon, in die Datenbank 3 eingespeist werden.

Der Datensatz 2A weist vorzugsweise therapierelevante bzw. fallbezogene Parameter bzw. Ergebnisparameter P_{AE} auf. Hierzu zählen insbesondere im laufenden Therapieablauf für den Patienten erhobene Daten. Dies kann sowohl Vitalparameter umfassen, die über entsprechende Sensoren gemessen werden können und, vorzugsweise, über den Eingabekanal 1 in Form eines Datensatzes 2A in die Datenbank 3 eingespeist werden, als auch beispielsweise Informationen über eine durchgeführte Untersuchung und/oder Behandlung in Form eines Berichts.

Mit der Therapiestation T₁ in Zusammenhang stehende, Maßnahmen betreffenden Parameter P_{AM} und/oder Ergebnisparameter P_{AE} werden insbesondere bei der Therapiestation T₁ erzeugt, im zu der Therapiestation T₁ korrespondierenden Datensatz 2A gespeichert und, vorzugsweise, über den Eingabekanal 1 in die Datenbank 3 eingespeist.

Der Datensatz 2A kann alternativ oder zusätzlich patientenspezifische bzw. personenbezogene Parameter P aufweisen. Zu diesen gehören insbesondere Patientenstammdaten, wie etwa der Name, Wohnort, der Kostenträger bzw. Versichertenstatus des Patienten oder aber bestimmte Vorerkrankungen oder sonstige Besonderheiten wie Allergien, Unverträglichkeiten oder ähnlichem.

Parameter P korrespondierend zu der zweiten und/oder weiteren Therapiestation T₂, T_{N} werden vorzugsweise über den Eingabekanal 1 in Form des weiteren Datensatzes 2B, 2N in die Datenbank 3 eingespeist.

Die zweite und/oder weitere Therapiestation T₂, T_{N} korrespondiert vorzugsweise zu einem anderen Zeitpunkt und/oder zu einem anderen Ort und/oder zu einer anderen Maßnahme als die erste Therapiestation T₁. Dies ist jedoch nicht zwingend. Es ist gleichermaßen möglich, dass zwei unterschiedliche Therapiestationen T₁, T₂, T_{N} demselben Zeitpunkt und/oder Ort zugeordnet sind und sich in funktionaler (maßnahmenbezogener) oder sonstiger Weise unterscheiden. Dies kann beispielsweise bei einer Blutuntersuchung der Fall sein, bei der nach einer einzelnen Probennahme im Rahmen einer gemeinsamen Analyse dieser Probe verschiedene Sätze von Blutwerten ermittelt werden, die charakteristisch sind für unterschiedliche Untersuchungsaspekte, wie beispielsweise die Bestimmung des Gerinnungsverhalten des Blutes und die Untersuchung auf Tumormarker.

Sind wenigstens zwei Datensätze 2A, 2B, 2N in der Datenbank 3 enthalten, so können diese über einen Auslesekanal 4 von einer bzw. durch eine Datenverarbeitungseinrichtung 5 gemeinsam oder einzeln ausgelesen, eingelesen und/oder abgerufen werden.

Ferner kann (jeweils) ein Rückkanal 4A vorgesehen sein, mittels dessen von der Datenverarbeitungseinrichtung 5 Datensätze 2 und/oder Parameter P, insbesondere in Richtung der Datenbank 3, übertragen und/oder in Datensätzen 2 abgelegt bzw. abgesichert werden können.

Vorzugsweise werden die Datensätze 2A, 2B, 2N bzw. Parameter P gemeinsam verarbeitet, um eine Ausgabe zu erlangen.

Im Darstellungsbeispiel weist die Ausgabe visuelle Komponenten auf. Hier sind jedoch auch andere Lösungen möglich.

Von der Datenverarbeitungseinrichtung 5 werden die Datensätze 2A, 2B, 2N vorzugsweise in einem oder mehreren Schritten, insbesondere gemeinsam, verarbeitet. Insbesondere werden die Parameter P der Datensätze 2A, 2B, 2N verrechnet, miteinander, mit Schwellwerten oder auf sonstige Weise verglichen oder gemeinsam verarbeitet. Hierdurch wird vorzugsweise ein Ergebnis erzeugt. Dieses Ergebnis korrespondiert bevorzugt zu einer Ausgabe oder einem Teil einer Ausgabe. Die Ausgabe kann insbesondere durch eine Benutzerschnittstelle 7 oder ein sonstiges Ausgabemittel ausgegeben werden.

Es versteht sich, dass auch lediglich einzelne Parameter P verschiedener Datensätze 2A, 2B, 2N von der Datenverarbeitungseinrichtung 5 verarbeitet werden können, d.h. nicht der vollständige Inhalt eines Datensatzes 2A, 2B, 2N muss in die Verarbeitung mit einfließen.

Die Benutzerschnittstelle 7 kann alternativ oder zusätzlich zur Ausgabe von Daten auch zur Eingabe von Daten, d.h. insbesondere Parametern P, ausgebildet sein. Insbesondere bei einer Therapiestation T₁, T₂, T_{N} jedoch auch zeitlich und/oder räumlich getrennt von dieser, insbesondere nachträglich, können die Therapiestation T₁, T₂, T_{N} betreffende Parameter P, insbesondere im Zusammenhang mit Maßnahmen der Therapiestation T₁, T₂ stehende Parameter P_{AM}, P_{BM}, T_{NM} und/oder Ergebnisparameter P_{AE}, P_{BE}, über die Benutzerschnittstelle 7 eingegeben und dem zur Therapiestation T₁, T₂, T_{N} korrespondierenden Datensatz 2A, 2B, 2N hinzugefügt werden.

Bei der Verarbeitung können auch weitere Datensätze 2N bzw. zu weiteren Therapiestationen T_{N} korrespondierende Parameter P berücksichtigt werden. Es ist hierbei bevorzugt, dass Parameter P korrespondierend zu, insbesondere einer, zwei, drei oder mehr, weiteren Therapiestationen T_{N} gemeinsam mit den Parametern korrespondierend zu der ersten und zweiten Therapiestationen T₁, T₂ oder hierzu korrespondierenden Datensätzen 2A, 2B verarbeitet werden.

Die Ausgabe über die Benutzerschnittstelle 7 umfasst dabei vorzugweise eine oder mehrere weitere Therapiestationen T₃, Optionen zur Freigabe oder Auswahl dieser und/oder eine Arbeitsanweisung und/oder eine Handlungsempfehlung korrespondierend hierzu.

Die Arbeitsanweisung und/oder eine Handlungsempfehlung an einen Benutzer kann einen medizinischen und/oder therapeutischen Bezug haben kann. Dementsprechend kann die Ausgabe, beispielsweise, Informationen und Anweisungen bzgl. der Medikation für einen Patienten umfassen und/oder die Art und Weise der Durchführung einer Untersuchung charakterisieren.

Beispielsweise können durchzuführende medizinische Untersuchungen oder sonstige Maßnahmen als weitere Therapiestation T₃ ausgegeben werden beispielsweise in Form einer To-do-Liste mit oder ohne zusätzlich angegebener Terminierung. Entsprechendes gilt für eine Anweisung bzw. Empfehlung bzgl. der Art und des Zeitpunkts der Durchführung einer oder mehrerer Maßnahmen.

Eine Terminierung, insbesondere von durchzuführenden Untersuchungen und/oder Behandlungen, betreffende Informationen können darüber hinaus auch gesondert oder in Kombination mit anderen Informationen ausgegeben werden.

Alternativ oder zusätzlich können auch weitere Kenngrößen K oder Parameter P ausgegeben werden, die nicht über die Benutzerschnittstelle 7 für einen menschlichen Benutzer angezeigt werden, sondern zur direkten Verarbeitung durch ein technisches Gerät, insbesondere ein medizinisch-therapeutisches und/oder ein medizinisch-diagnostisches Gerät, in maschinenlesbarer Form bereitgestellt bzw. an ein solches Gerät übermittelt werden.

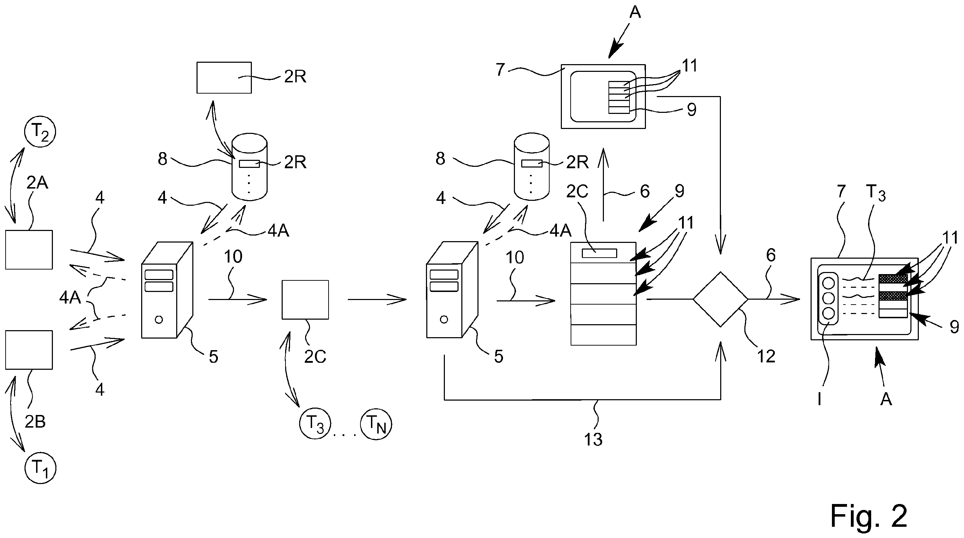

Ein beispielhafter Weg, auf dem mittels des vorschlagsgemäßen Verfahrens zu einer Ausgabe durch die Benutzerschnittstelle 7 auf Basis zweier Datensätze 2 gelangt wird, ist in Fig. 2 schematisch wiedergegeben.

Zwei Datensätze 2 werden dabei zunächst aus einer nicht dargestellten Datenbank 3 über Auslesekanäle 4 von einer bzw. durch eine Datenverarbeitungseinrichtung 5 ausgelesen.

Zusätzlich zu den Datensätzen 2A, 2B aus der Datenbank 3 kann die Datenverarbeitungseinrichtung 5 über einen weiteren Auslesekanal 4 einen oder mehrere Datensätze 2 aus einer zusätzlichen Quelle auslesen. In vorliegender Darstellung wird die zusätzliche Datenquelle als externe Datenbank 8 dargestellt.

Die externe Datenbank 8 kann insbesondere Datensätze 2R enthalten, die vorzugsweise nicht zu Therapiestationen T₁, T₂, T₃ eines Therapieablaufs für einen Patienten korrespondieren. Solche Datensätze 2R können beispielsweise die Verfügbarkeit von Ressourcen oder interne Regeln zum Ablauf in einer medizinisch-therapeutischen Einrichtung betreffen.

Bevorzugt werden durch die zusätzliche Datenquelle, d. h. vorliegend durch die externe Datenbank 8, zudem Datensätze 2R mit Sollwertvorgaben für messbare Parameter P bereitgestellt.

In einem Verarbeitungsschritt wendet die Datenverarbeitungseinrichtung 5 auf die Datensätze 2A, 2B, 2N, 2R aus der Datenbank 3 und ggf. aus der externen Datenbank 8 als zusätzlicher Quelle, vorzugsweise, einen Algorithmus an, der insbesondere aus einer Mehrzahl von Teilalgorithmen bestehen kann.

Als Argumente für den Algorithmus dienen dabei die Parameter P aus den Datensätzen 2A, 2B, 2N, 2R, die von der Datenverarbeitungseinrichtung 5 ausgelesen wurden.

Ein Algorithmus kann in der Anwendung einer Rechenoperation bestehen, insbesondere falls es sich bei den Parametern P um Zahlenwerte handelt. Ferner lassen sich die Parameter P sortieren. Möglich ist zudem der Vergleich der Parameter P miteinander und/oder mit Vorgabewerten. Insbesondere werden Parameter P unterschiedlicher der Datensätze 2A, 2B, 2R miteinander verglichen, verrechnet, bewertet und/oder verarbeitet.

Darüber hinaus ist es bevorzugt, dass ausgehend von den Datensätzen 2A, 2B mittels der Datenverarbeitungseinrichtung 5, vorzugsweise durch eine oder mehrere numerische Methoden, eine Parameterentwicklung simuliert wird. Dies kann insbesondere die zeitliche Entwicklung der Parameter P betreffen. Ferner kann jedoch auch ein Zusammenwirken verschiedener Parameter P unter Bedingungen simuliert werden, die zumindest teilweise dem Zufall unterliegen. Das letztgenannte Beispiel kann insbesondere Bedeutung für eine Risikobewertung haben.

Vorzugsweise kann mittels der Datenverarbeitungseinrichtung 5, vorzugsweise durch eine Simulation, insbesondere wenigstens ein Parameter P eines Datensatzes 2C, 2N einer zukünftigen Therapiestation T₃, T_{N} ermittelt werden. Dabei kann es sich insbesondere um einen mit der Therapiestation T₃, T_{N} verbundene Maßnahmen betreffenden Parameter P_{3M}, P_{NM} und/oder einen ein bei der, durch die oder im Zusammenhang mit der Therapiestation T₃, T_{N} zu erreichendes oder erreichbares Therapieziel bzw. -ergebnis betreffenden Ergebnisparameter P_{3F}, P_{NE} handeln.

Durch die Anwendung eines Algorithmus auf die Datensätze 2 erzeugt die Datenverarbeitungseinrichtung 5 vorzugsweise ein Ergebnis 9, das über einen internen Ausgabekanal 10 zunächst grundsätzlich im erfindungsgemäßen System S S bzw. für das erfindungsgemäße Verfahren bereitgestellt und/oder ganz oder teilweise ausgegeben oder weiterverarbeitet werden kann.

Das Ergebnis 9 ist insbesondere ein Satz einer Mehrzahl von Optionen 11. Jede Option 11 betrifft und/oder umfasst, vorzugsweise, jeweils einen Datensatz 2, der zu einer möglichen Therapiestation T₃, T_{N} korrespondiert. Durch die Optionen 11 des Ergebnisses 9 können dementsprechend verschiedene Therapiemöglichkeiten für einen Patienten repräsentiert werden.

Es versteht sich, dass der interne Ausgabekanal 10 in einen Ausgabekanal 6 zu einer Benutzerschnittstelle 7 übergehen kann, durch die das Ergebnis 9 in Form einer Ausgabe ganz oder teilweise wiedergegeben wird.

Es ist jedoch auch möglich, dass das Ergebnis 9 in einem weiteren Verarbeitungsschritt, insbesondere von der Datenverarbeitungseinrichtung 5, weiterverarbeitet wird. Hierbei können grundsätzlich weitere Datensätze 2A, 2B aus der Datenbank 3 und/oder aus einer zusätzlichen Quelle, beispielsweise aus einer externen Datenbank 8, einbezogen werden. Die Datenverarbeitungseinrichtung 5 kann bei diesem weiteren Verarbeitungsschritt einen weiteren Algorithmus auf den oder die Datensätze 2 und/oder das Ergebnis 9 anwenden. Dabei muss der Algorithmus insbesondere nicht dem Algorithmus des ersten Verarbeitungsschritts entsprechen.

Die Durchführung des weiteren Verarbeitungsschritts durch die Datenverarbeitungseinrichtung 5 führt vorzugsweise dazu, dass über den internen Ausgabekanal 10 ein Ergebnis 9 ausgegeben wird.

Das Ergebnis 9 kann direkt über den Ausgabekanal 6 durch eine Benutzerschnittstelle 7 ausgegeben und/oder von der Datenverarbeitungseinrichtung 5 in einem weiteren Verarbeitungsschritt weiterverarbeitet werden kann.

Die Verarbeitung kann dabei insbesondere iterativ und/oder in mehreren Verarbeitungsschritten erfolgen.

Im vorliegenden Ausführungsbeispiel weist das Ergebnis 9 des zweiten Verarbeitungsschritts eine Mehrzahl von Optionen 11 auf, die insbesondere in Form von Therapiestationen T₃ mögliche Ausgestaltungen einer weiteren oder folgenden Therapie eines Therapieablaufs eines Patienten betreffen können.

Das Ergebnis 9 mit den Optionen 11 kann nun unverändert ganz oder teilweise über einen Ausgabekanal 6 durch eine Benutzerschnittstelle 7, insbesondere visualisierst, ausgegeben werden.

Alternativ oder zusätzlich kann die Benutzerschnittstelle 7 ein Freigabemittel 12 aufweisen. Mittels des Freigabemittels 12 kann die Ausgabe durch die Benutzerschnittstelle 7 beeinflusst werden. Durch das Freigabemittel 12 können beispielsweise einzelne, mehrere oder alle Optionen 11 bzw. Therapiestationen T₃ ausgewählt, selektiert, und/oder freigegeben werden. Hierzu wird vorzugsweise ein Freigabesignal 13 mittels oder durch Bedienung des Freigabemittels 12 erzeugt, wodurch eine oder mehrere der Optionen ausgewählt, freigegeben, bestätigt und/oder gelöscht, verworfen oder deselektiert werden können.

In einer besonders bevorzugten Ausführungsform bildet die Benutzerschnittstelle 7 das Freigabemittel 12, beispielsweise durch eine Taste, ein Feld auf einem berührungsempfindlichen Anzeigemittel oder durch ein sonstiges Eingabegerät.

Das Freigabemittel 12 ist vorzugsweise einer Option 11 bzw. hierzu korrespondierenden Therapiestation T₃ zugeordnet. Auf diese Weise kann durch das Freigabemittel 12 die Option(en) 11 bzw. Therapiestation(en) T₃ ausgewählt, freigegeben, bestätigt, gelöscht, verworfen und/oder deselektiert werden. Ein hierzu korrespondierendes Freigabesignal 13 wird vorzugsweise durch Bedienung des Freigabemittels 12 erzeugt und dazu verwendet, Optionen 11 bzw. Therapiestationen T₃ als ausgewählt bzw. verworfen zu markieren. Im Folgenden ist es möglich, das nur noch ausgewählte bzw. freigegebene Optionen 11 bzw. Therapiestationen T₃ ausgeben bzw. durch eine (andere) Benutzerschnittstelle 7 zur Verfügung gestellt werden, insbesondere zur Auswahl und/oder Definition zusätzlicher Kenngrößen oder Parameter, die zu der jeweiligen Option 11 bzw. Therapiestation T₃ korrespondieren. Beispiele sind hierbei Parameter, die zu Orten, Zeiten oder allgemein Terminen der jeweiligen Therapiestation T₃ oder Optionen 11 korrespondieren. Vorzugsweise ist das System S so ausgebildet, bei Erzeugung des Freigabesignals 13 über das Freigabemittel 12 die hierzu korrespondierende Option 11 bzw. Therapiestation T₃ in entsprechender Weise zu markieren. Insbesondere erfolgt dies durch oder in der Datenverarbeitungseinrichtung 5 und/oder durch Ablegen, Ändern und/oder Hinzufügen eines entsprechenden Parameters in den jeweiligen Datensatz 2C der zu der Therapiestation T₃ und/oder der Option 11 korrespondiert.

Das Freigabesignal 13 kann dabei insbesondere von der Datenverarbeitungseinrichtung 5, vorzugsweise automatisch, übermittelt werden, nachdem beispielsweise zusätzliche Parameter P ausgelesen und einbezogen wurden. Diese zusätzlichen Parameter P können aus einer zusätzlichen Quelle, beispielsweise einer externen Datenbank 8 und/oder einem Ressourcenmanagementsystem stammen. Je nach Eigenschaften der zusätzlichen Parameter P, die beispielsweise eine Ressourcenverfügbarkeit betreffen können, kann das Freigabesignal 13 derart konfiguriert werden, dass einzelne oder alle Optionen 11 von der Ausgabe durch die Benutzerschnittstelle 7 ausgeschlossen werden. Bei dem Beispiel einer zusätzlich miteinbezogenen Ressourcenverfügbarkeit kann ein solcher Ausschluss insbesondere mangels verfügbarer Ressourcen erfolgen.

Alternativ oder zusätzlich zu einer automatischen Freigabe durch die Datenverarbeitungseinrichtung 5 mittels eines Freigabesignals 13 kann die Freigabe auch durch ein Freigabesignal 13 erfolgen, das durch eine Eingabe über die Benutzerschnittstelle 7 erzeugt wird. In diesem Fall ist es bevorzugt, dass das Ergebnis 9 zunächst mit allen Optionen 11 bzw. Therapiestationen T₃, T_{N} durch eine bzw. dieselbe Benutzerschnittstelle 7 ausgegeben wird.

Nach einer manuellen Auswahl, beispielsweise durch medizinisch fachkundiges Personal, insbesondere einen Arzt und/oder nach therapeutischen Gesichtspunkten, können einzelne Optionen 11 mittels einer Eingabe über die Benutzerschnittstelle 7 ausgewählt und durch Übermittlung des Freigabesignals 13 freigegeben werden. In Folge dessen wird vorzugsweise durch eine andere Benutzerschnittstelle 7, insbesondere bei einer weiteren Therapiestation, nur ein Teil der Optionen 11 des ursprünglichen Ergebnisses 9 ausgegeben.

Die Ausgabe, bei der es sich um eine Arbeitsanweisung und/oder einer Handlungsempfehlung an einen Benutzer handeln kann, ist durch das erfindungsgemäße Verfahren an die betreffende Therapiestation angepasst.

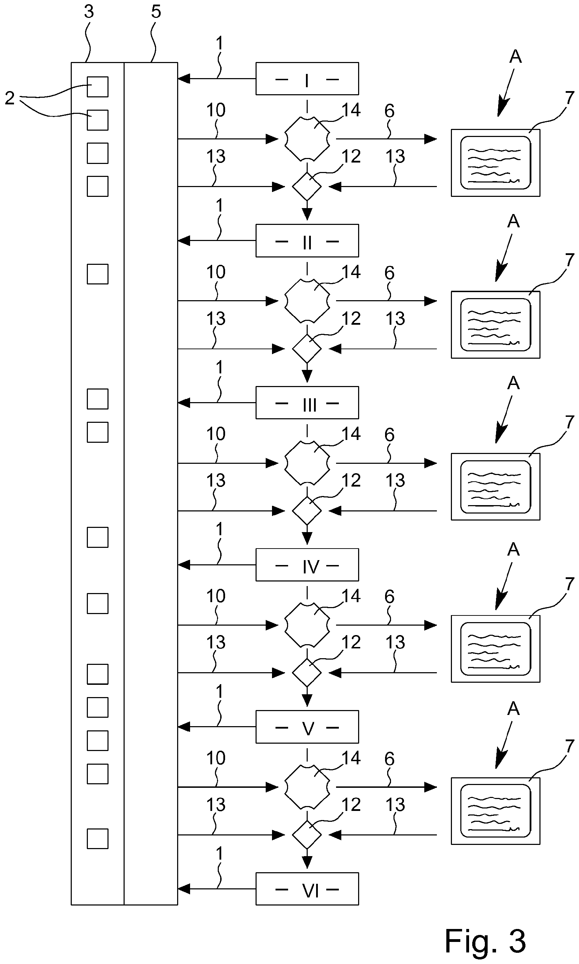

In Fig. 3 ist schematisch ein möglicher, durch das erfindungsgemäße Verfahren unterstützter Therapieablauf für einen Patienten dargestellt. Der Therapieablauf ist in der vorliegenden Darstellung in sechs Phasen I bis VI gegliedert. Die Phasen I bis VI korrespondieren jeweils zu unterschiedlichen Therapiestationen T₁, T₂, T₃, T_{N} bzw. Behandlungsabschnitten des Therapieablaufs.

Eine Phase I-VI umfasst insbesondere einen stationären Aufenthalt in einer medizinisch-therapeutischen Einrichtung, wie beispielsweise einer Akutklinik und/oder einer Reha-Einrichtung. Unterschiedliche Phasen I-VI unterscheiden sich folglich bevorzugt in Bezug auf die Einrichtung, die die Therapiestationen T₁, T₂, T₃, T_{N} durchführt und/oder der die Therapiestationen T₁, T₂, T₃, T_{N} zugeordnet sind.

In einem konkreten Beispiel kann Phase I sich auf eine ambulante Behandlung durch einen niedergelassenen Arzt beziehen, an die sich eine klinische Akutbehandlung als Phase II anschließt. Phase III kann beispielsweise in Form einer Aktivierungspflege dazu dienen, den Patienten für eine stationäre Rehabilitation als Phase IV ausreichend zu mobilisieren. An die stationäre Rehabilitation kann sich als Phase V eine Fortsetzung der Rehabilitation im ambulanten Bereich anschließen. Die abschließende Phase VI kann sich auf eine allgemeine Nachsorge im häuslichen und/oder ambulanten Bereich beziehen.

Die Phasen können auch mit anderem bzw. abweichendem Inhalt, in einer anderen Reihenfolge, Zusammenstellung oder relativen Anordnung zueinander vorgesehen sein. Insbesondere kann eine Wiederholung von einer oder mehrerer Phasen und/oder eine andere Anzahl als die dargestellten sechs Phasen vorgesehen sein.

Jede der Phasen I bis VI stellt als solche vorzugsweise selbst eine Therapiestation T₁, T₂, T₃, T_{N} dar, kann jedoch auch mehrere Therapiestationen T₁, T₂, T₃, T_{N} enthalten.

Als Therapiestation T₁, T₂, T₃, T_{N} im Sinne der Erfindung kann letztlich jeder räumlich, zeitlich und/oder funktional abgrenzbare Teilabschnitt des Therapieablaufs für einen Patienten verstanden werden.

Zwischen einzelnen Therapiestationen T₁, T₂, T₃, T_{N}, insbesondere zwischen den einzelnen Phasen I bis VI, können jeweils Schnittstellen 14 bestehen. An jeder Schnittstelle 14 wird vorzugsweise von einer Therapiestation T₁, T₂, T₃, T_{N} zu einer anderen Therapiestation T₁, T₂, T₃, T_{N} übergeleitet.

Um den Therapieablauf als solchen weiterzuführen, ist es beim Übergang von einer Therapiestation T₁, T₂, T₃, T_{N} zu einer anderen, insbesondere von einer Phase I-VI zur nächsten, bevorzugt, dass zum einen einer oder mehrere der Parameter P der einen Therapiestation T₁, T₂, T₃, T_{N}, an die andere Therapiestation T₁, T₂, T₃, T_{N}, insbesondere unverändert, weitergegeben werden. Dies kann beispielsweise eine begonnene und weiterzuführende Behandlung und/oder Medikation für einen Patienten betreffen.

Ferner ist es bevorzugt, dass an der Schnittstelle 14 vor einer Therapiestation T₁, T₂, T₃, T_{N} erforderliche Informationen bereitstehen, die speziell diese Therapiestationen T₁, T₂, T₃, T_{N} betreffen.

Für die dritte bzw. folgende Therapiestation T₃, T_{N} erforderliche Informationen der vorgenannten Art können insbesondere Parameter P sein, die für die nachfolgende Therapiestation T₃, T_{N} bzw. Phase I-VI erforderliche Ressourcen und/oder durchzuführende Untersuchungen und/oder Behandlungen betreffen.

Zur Bereitstellung der erforderlichen Informationen an der, mit der oder durch die Schnittstelle 14 werden vorzugsweise in wenigstens einer vorhergehenden und/oder der aktuellen Phase I-VI bzw. an wenigstens einer vorhergehenden und/oder der aktuellen Therapiestation T₁, T₂, T_{N} zu der Phase I-VI bzw. Therapiestation T₁, T₂, T_{N} jeweils korrespondierende Datensätze 2 insbesondere durch Erfassung von Parametern P erzeugt und gespeichert.

Die Datensätze 2 können zum einen Parameter P enthalten, die rein zum Zweck der Dokumentation des Therapieablaufs bzw. der Therapiestation T₁, T₂, T₃, T_{N} in der Datenbank 3 abgelegt werden.

Parameter P, die von Bedeutung sind für die weitere Ausgestaltung des Therapieablaufs, d. h. insbesondere für eine nachfolgende Phase I-VI und/oder Therapiestation T₃, T_{N}, werden vorzugsweise von der Datenverarbeitungseinrichtung 5 ausgewählt, abgerufen und/oder entsprechend verarbeitet, um die an der Schnittstelle 14 insbesondere vor einer nachfolgenden Phase I-VI, für diese Phase I-VI erforderlichen Informationen in Form einer Ausgabe durch die Benutzerschnittstelle 7 bereitzustellen.

Ein Benutzer, der im Zusammenhang mit der betreffenden Phase I-VI des Therapieablaufs für einen Patienten tätig ist, kann der Ausgabe durch die Benutzerschnittstelle 7 eine entsprechende Handlungsempfehlung bzw. -anweisung entnehmen, die beispielsweise die wesentlichen durchzuführenden Vorbereitungsmaßnahmen für die betreffende Phase I-VI umfasst.

Handelt der Benutzer gemäß der ausgegebenen Handlungsempfehlung, gewährleistet dies an der Schnittstelle 14 einen nahtlosen Übergang von einer Phase zur nächsten Phase, bzw. innerhalb einer Phase von einer Therapiestation T₁, T₂, T₃, T_{N} zu einer anderen Therapiestation T₃, T_{N}. Insbesondere Fehler, die auf eine unvollständige Dokumentation des bisherigen Therapieablaufs und/oder eine lückenhafte Kommunikationskette zurückzuführen sind, werden auf diese Weise erfolgreich vermieden.

Die Handlungsempfehlung kann insbesondere verschiedene Optionen 11 umfassen, die in Abhängigkeit von weiteren Parametern P, insbesondere von einer Ressourcenverfügbarkeit, durch das Freigabemittel 12 für die jeweils nachfolgende Phase freigegeben werden.

Dazu kann von der Datenverarbeitungseinrichtung 5, beispielsweise nach einem Abgleich mit Datensätzen 2A, 2B, 2R von einer Datenbank 8 und/oder einem Ressourcenmanagementsystem, ein entsprechendes Freigabesignal 13 durch das Freigabemittel 12 übermittelt werden, wobei die Übermittlung des Freigabesignals 13 insbesondere automatisch erfolgt.

Alternativ oder zusätzlich können zunächst entsprechend der Darstellung in Fig. 2 alle Optionen 11 als Therapievorschläge über die Benutzerschnittstelle 7 ausgegeben werden.

Nach einer Auswahl, beispielsweise durch einen Arzt und/oder fachlichen Experten für die betreffende, d. h. in der Regel für die nachfolgende, Phase I-VI, können mittels einer Eingabe über die Benutzerschnittstelle 7 eine einzelne, mehrere oder auch alle Optionen 11 durch Übermittlung des entsprechenden Freigabesignals 13 durch das Freigabemittel 12 von diesem freigegeben werden.

Vorzugsweise werden zur Bereitstellung der erforderlichen Informationen an der Schnittstelle 14 vor einer Phase I-VI nicht zwingend ausschließlich Parameter P der vorhergehenden bzw. der aktuellen Phase I-VI einbezogen. Vielmehr werden vorzugsweise aus der Gesamtheit aller Datensätze 2 in der Datenbank 3, d. h. aus allen an vorhergehenden Therapiestationen T₁, T₂ ,T_{N} erzeugten und gespeicherten Datensätze 2, diejenigen ausgewählt und verarbeitet, die vom System S bzw. Datenverarbeitungseinrichtung 5 als bedeutsam für die nachfolgende Phase I-VI erkannt werden. Dies ist ein wesentlicher Unterschied der Erfindung gegenüber bekannten Verfahren, bei denen eine reine Dokumentation bzw. Bereitstellung von gespeicherten Datensätzen 2 erfolgt.

Zudem zeichnet sich das System S bzw. das Verfahren durch Ermittlung, Simulation und/oder Prognostizierung von Eigenschaften wenigstens eine nachfolgende Phase I-VI und/oder Therapiestation durch eine Zielorientierung aus. Im Gegensatz dazu sind bekannte Systeme, wie beispielsweise ein übliches Krankenhausinformationssystem, insbesondere auf eine gegenwärtige bzw. rückgerichtete Dokumentation eines Therapieablaufs ausgerichtet.

Die einzelnen Phasen I bis VI des Therapieablaufs für einen Patienten müssen nicht zwingend zeitlich streng aufeinanderfolgend ablaufen, obwohl dies überwiegend bevorzugt ist.

Es trägt im Gegenteil zu einem einheitlichen Therapieablauf mit den Vorteilen besserer Erfolgsaussichten für die Therapie sowie geringeren Kostenaufkommen bei, wenn Teilaspekte der Phasen I-VI insbesondere zeitlich parallel ablaufen oder ablaufen können. Dies wird in vorteilhafter Weise durch das erfindungsgemäße Verfahren bzw. das erfindungsgemäße System S erleichtert oder überhaupt erst ermöglicht.

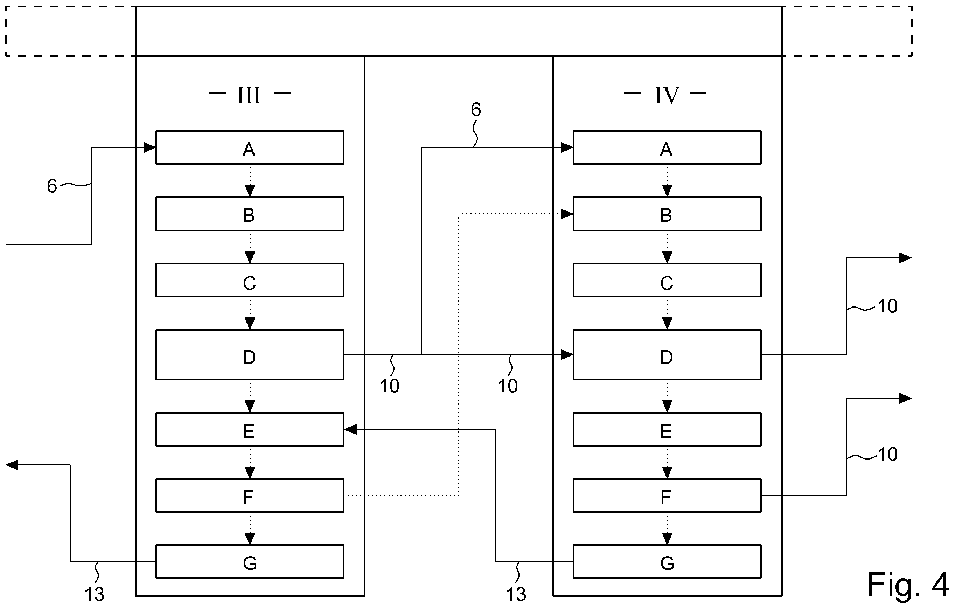

In Fig. 4 sind beispielhaft zwei Phasen III, IV eines Therapieablaufs mit verschiedenen Teilaspekten A bis G jeder Phase III, IV dargestellt. Die Phasen III, IV können beispielsweise einerseits den Aufenthalt in einer Akutklinik und andererseits in einer stationären Rehabilitations-Einrichtung betreffen. Jede der Phasen umfasst in der vorliegenden Darstellung, mehrere, eine Abfolge oder Reihe funktionaler Teilaspekte oder Phasenabschnitte A bis G, die vorzugsweise Therapiestationen T₁, T₂, T₃, T_{N} sind oder aufweisen.

Falls möglich, ist jeder Phase I-VI eine Vorbereitung A vorangestellt, damit die Abläufe der betreffenden Phase I-VI möglichst ohne große zeitliche Verzögerung erfolgen können. Hierbei kann es sich um eine Therapiestation T₁ zur Ermittlung von Vitalparametern o. dgl. handeln. Hierbei können Parameter P ermittelt und als bzw. in Form des Datensatzes 2A abgelegt bzw. abgespeichert werden.

Die Parameter P können darüber hinaus auch rein organisatorischer Art sein. Insbesondere muss ein Maßnahmen der Therapiestation T₁ betreffender Parameter P_{AM} nicht ausschließlich einen medizinisch-therapeutischen Charakter aufweisen, wie es beispielsweise bei Vitalwerten oder einer ICD- bzw. ICF-Klasse der Fall ist, sondern kann sich allgemein auf eine für den Therapieablauf eines Patienten relevante Maßnahme beziehen.

Letztlich kann auch ein formaler Vorgang, wie die organisatorische Vorbereitung des Therapieablaufs eine Therapiestation T₁ im Sinne der Erfindung darstellen.

Nach Ablauf der Vorbereitung A erfolgt im nächsten Schritt die Aufnahme B des Patienten in die betreffende medizinisch-therapeutische Einrichtung, insbesondere als oder in Form einer zweiten Therapiestation T₂. Hier können ergänzende Vitalparameter oder sonstige Parameter P ermittelt werden und/oder in Form des Datensatzes 2B abgelegt bzw. abgespeichert werden.

Vorschlagsgemäß kann durch eine gemeinsame Verarbeitung, insbesondere ein Vergleich, der Parameter P der Datensätze 2A, 2B ein weiterer Phasenabschnitt AG bzw. der Inhalt des weiteren Phasenabschnitts A-G, insbesondere in Form einer Therapiestation T₃ ermittelt werden.

Hierzu können die Parameter P der ersten Therapiestation T₁ und der zweiten Therapiestation T₂ miteinander verglichen, miteinander verrechnet oder auf sonstige Weise miteinander und/oder mit weiteren Parametern P, beispielsweise korrespondierend zu benötigten Ressourcen oder hierzu korrespondierender Datensätze 2R, ermittelt werden.

Beispielsweise kann das Ergebnis 9 eine oder mehrere mögliche folgende Therapiestationen T₃ sein oder aufweisen, im Darstellungsbeispiel in Form einer Behandlung C bzw. Optionen 11 für weitere, folgende Therapiestationen T₃, die zu der Behandlung C korrespondieren oder der Behandlung C entsprechen.

Vorzugsweise wird der Patient nachfolgend der Behandlung C unterzogen. Hierbei kann der Patient, sein Assistent, eine Hilfsperson, der Arzt, die Geräte o. dgl. mittels der Benutzerschnittstelle 7 zu und/oder durch die Behandlung C bzw. die hierzu korrespondierende Therapiestation T₃ geführt werden. Zu diesem Zweck kann die Benutzerschnittstelle angaben zu Ort und Zeit der Behandlung C bzw. der Therapiestation T₃ visualisieren oder auf sonstige Weise ausgeben, so dass der Patient sich am Ort der Behandlung C bzw. Therapiestation T₃ einfinden kann und/oder die bei der Behandlung C bzw. Therapiestation T₃ anstehenden Maßnahmen spezifiziert werden können.

Die Benutzerschnittstelle 7 kann zu diesem Zweck mehrteilig ausgebildet sein, so dass koordinatorische Anweisungen wie Ort, Zeit ggfs. weitere Randbedingungen wie nüchternes Erscheinen, besondere Vorbereitungen o. dgl. angegeben werden. In einer anderen oder einem anderen Teil der Benutzerschnittstelle 7 können dem medizinischen Personal Detailinformationen bezüglich der durchzuführenden Behandlung C bzw. Therapiestation T₃ visualisiert oder übermittelt werden und/oder einem Gerät oder einer Einrichtung für die Behandlung C bzw. Therapiestation T3 können Daten wie Arbeitseinstellung o. dgl. zugeführt werden. Die Benutzerschnittstelle 7 kann also durch mehrere, voneinander getrennte Geräte gebildet sein.

Zur Behandlung C gehören beispielsweise notwendige Untersuchungen und/oder medizinisch-therapeutische Maßnahmen. Letztere umfassen im Akutbereich insbesondere durchgeführte Operationen und/oder eine Medikation für den Patienten. Im Fall einer stationären Rehabilitationsbehandlung kann dies beispielsweise Anwendungen und Übungen sowie einen speziellen Ernährungsplan oder ähnliches umfassen.

Ist die Behandlung C in einer Einrichtung soweit abgeschlossen, dass die Weiterbehandlung entsprechend der nächsten Phase IV im Therapieablauf bevorsteht, so wird diese Weiterbehandlung vorzugsweise erneut mit dem vorschlagsgemäßen Verfahren vorbereitet.

Insbesondere ist es möglich, dass zu den vorhergehenden Therapiestationen T₁, T₂ und/oder T₃ korrespondierende Parameter P bzw. Datensätze 2A, 2B, 2C analysiert, gemeinsam verwendet, verrechnet, verglichen oder auf sonstige Weise verarbeitet werden, um den funktionalen Teilaspekt D zu ermitteln, insbesondere einen oder mehrere weitere Therapiestationen T_{N} und/oder in Form einer Weiterbehandlungsempfehlung D, die beispielsweise im Rahmen eines Abschlussberichts durch den leitenden Arzt abgegeben werden kann.

Vorzugsweise werden eine oder mehrere Weiterbehandlungsempfehlungen D automatisch vom erfindungsgemäßen System S anhand von im Laufe des Therapieablaufs gespeicherten Datensätzen 2 vorgeschlagen.

Durch eine Anbindung beider, den dargestellten Phasen III, IV zugeordneten medizinisch-therapeutischen Einrichtungen an das erfindungsgemäße System S kann die Weiterbehandlungsempfehlung D in Form eines Ergebnisses 9, insbesondere mit einer oder einer Mehrzahl von (bereits freigegebenen oder noch freizugebenden) Optionen 11 über den internen Ausgabekanal 10 und/oder den Ausgabekanal 6 zur Bestimmung bzw. inhaltlichen Definition der nächsten Phase IV übermittelt werden.

Dort kann beispielsweise durch die Benutzerschnittstelle 7 eine Handlungsempfehlung und/oder Arbeitsanweisung zur Vorbereitung A der bevorstehenden Phase IV ausgegeben werden. Alternativ oder zusätzlich kann eine Freigabe wie zuvor beschrieben erfolgen. Es ist weiter bevorzugt, dass sowohl in der laufenden als auch für die folgende Phase Freigaben oder Teilfreigaben und/oder gemeinsame Freigabeprozesse vorgesehen sind.

Möglich ist darüber hinaus auch, dass nach Übermittlung eines die Weiterbehandlung D betreffenden Datensatzes 2 weitere, die bevorstehende Phase IV betreffende Parameter P einbezogen und mit dem die Weiterbehandlungsempfehlung D betreffenden Datensatz 2 verarbeitet werden, um erst danach zu einer Ausgabe durch die Benutzerschnittstelle 7 zu gelangen.

Zur Vorbereitung A einer Phase III, IV werden vorzugsweise, nicht allein Parameter P der vorhergehenden Phase zugrunde gelegt.

Durch die Anbindung aller zu den verschiedenen Phasen I-VI des Therapieablaufs zugehörigen Einrichtungen an das erfindungsgemäße System S bzw. die Anwendung des Verfahrens auf den gesamten Therapieablauf für einen Patienten ist es insbesondere möglich, dass auch Parameter P von weiter zurückliegenden und/oder mehreren, beispielsweise mehr als 2, 3 oder 4 Phasen I-VI bzw. Therapiestationen T₁, T₂, T₃, T_{N} in die über die Benutzerschnittstelle 7 ausgegebene Arbeitsanweisung zur Vorbereitung A einer Phase I-Vlmit einfließen.

Entsprechendes gilt für die Weiterbehandlungsempfehlung D. Wird diese in Form eines oder mehrerer Optionen 11 ausgegeben, wobei die Optionen 11 insbesondere Therapievorschläge bzw. mögliche Therapiestationen T₁, T₂, T₃, T_{N} sein können, so kann vorgesehen sein, dass zur Ermittlung des Ergebnisses 9 mit den Optionen 11 auch Parameter P mit einbezogen werden können, die zu Therapiestationen T₁, T₂, T₃, T_{N} weiter zurückliegender Phasen I-VI korrespondieren.

Mit anderen Worten kann eine weitere Therapiestation T₁, T₂, T₃, T_{N} für eine der folgenden Phasen I-IV noch während des Verlaufs der aktuellen Phase III ermittelt werden. Dies ermöglicht es, weitere Therapiestationen T₁, T₂, T₃, T_{N} der aktuellen Phase so zu definieren, dass ein reibungsloser Übergang zwischen den Phasen I-VI gewährleistet werden kann.

Beispielsweise wird aufgrund von Parametern P bzw. Datensätzen 2A, 2B von bereits erfolgten Therapiestationen T₁, T₂, aus der laufenden Phase III oder früheren Phasen I, II eine oder mehrere Therapiestationen T₃ bzw. ein Verlauf dieser, insbesondere in Form der folgenden Phase IV ermittelt, beispielsweise als Weiterbehandlungsempfehlung D.

Daraufhin kann der weitere Verlauf der aktuellen Phase III, beispielsweise charakterisiert durch weitere Therapiestationen T₃, so gestaltet werden, dass der Übergang zwischen der aktuellen und der folgenden Phase I-VI möglichst reibungslos bewerkstellig werden kann. Es ist also insbesondere möglich, das in Abhängigkeit von den ermittelten Therapiestationen T₃, T_{N} der folgenden Phase IV Therapiestationen T₃, T_{N} ermittelt und definiert werden, die vor Abschluss der laufenden Phase III noch durchgeführt werden.

Vorzugsweise erfolgt die Weiterbehandlungsempfehlung D für die nächste Phase IV möglichst frühzeitig, insbesondere während des Aufenthalts des Patienten in der der aktuellen Phase III zugeordneten medizinisch-therapeutischen Einrichtung und/oder Therapiestation T₁, T₂, T_{N}. Durch eine Ausgabe über einer Benutzerschnittstelle 7 im Bereich der der nachfolgenden Phase IV zugeordneten Einrichtung wird ermöglicht, dass die Vorbereitung A der nachfolgenden Phase IV unter Berücksichtigung von Parametern P, welche im Zusammenhang mit Therapiestationen T₁, T₂, T_{N} der aktuellen bzw. vorhergehenden Phase III, insbesondere der Weiterbehandlungsempfehlung D, stehen, rechtzeitig beginnen bzw. abgeschlossen werden kann, um eine Freigabe E für eine Entlassung F des Patienten aus der aktuellen Einrichtung bzw. Phase zu bewirken.

Auf die Entlassung F aus der Einrichtung der einen Phase III folgt vorzugsweise unmittelbar oder mit dem kleinsten aus medizinischer Sicht sinnvollen zeitlichen Abstand die Aufnahme B in der der nachfolgenden Phase IV zugeordneten medizinisch-therapeutischen Einrichtung, beispielsweise einer stationären Reha-Einrichtung.

Nach der Entlassung F des Patienten werden Ressourcen, die im Zusammenhang mit der Behandlung des Patienten belegt waren im Sinne einer Ressourcenfreigabe G wieder bereitgestellt.

Über ein entsprechendes Freigabesignal 13 kann diese Ressourcenfreigabe G insbesondere Einfluss auf die Freigabe E als Teilaspekt einer vorhergehenden Phase III haben.

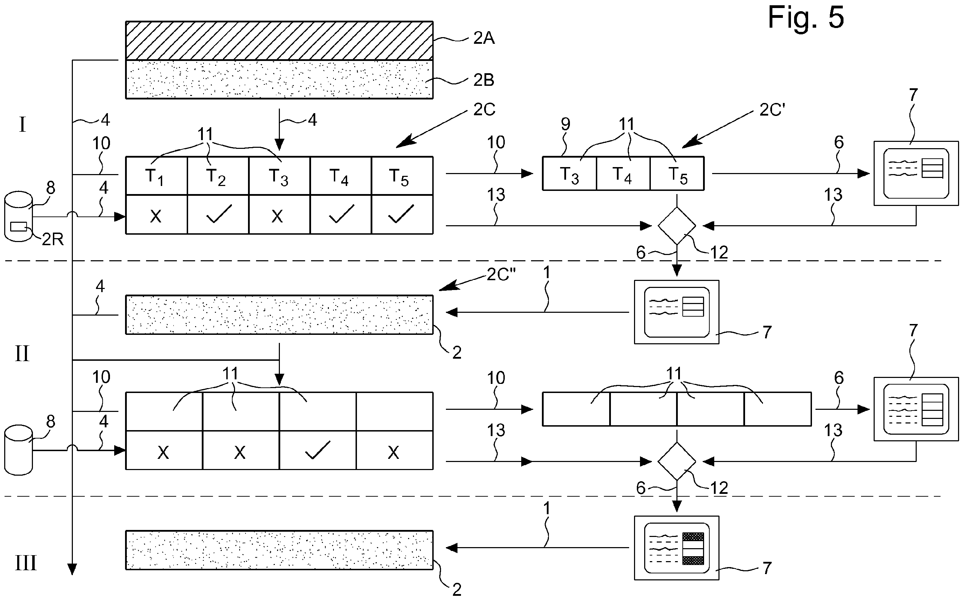

In Fig. 5 ist eine alternative Darstellung eines Aspekts des vorschlagsgemäßen Verfahrens dargestellt. In der vorliegenden Darstellung ist in schematischer Weise erkennbar, wie die Datenverarbeitung über mehrere Phasen I-VI hinweg erfolgen kann. Der in Fig. 5 dargestellte Ablauf lässt sich in einen internen Teil, in vorliegender Darstellung auf der linken Seite, sowie einen nach außen gerichteten Teil, d. h. einen Teil mit einer Außenwirkung, in der Darstellung gemäß Fig. 5 auf der rechten Seite, unterteilen.

Der interne Teil betrifft dabei die Speicherung und Verarbeitung der Datensätze 2 mittels der Datenbank 3 bzw. der Datenverarbeitungseinrichtung 5. Diesem systeminternen Teil lassen sich alle Prozesse zuordnen, die weder mit der Eingabe noch mit der Ausgabe von Therapiestationen T₁, T₂, T₃, Parametern P, Optionen 11 und/oder Datensätzen 2 in direkter Beziehung stehen.

Dem nach außen gerichteten Teil werden im Gegenzug diejenigen Verfahrensschritte bzw. Systembestandteile zugeordnet, über die letztlich entweder vom System S Einfluss auf den Therapieablauf für einen Patienten genommen wird oder von einem Benutzer und/oder aufgrund von wenigstens einem therapierelevanten Ereignis Einfluss auf das Verfahren bzw. das System S genommen wird. Dies betrifft insbesondere die Eingabe therapierelevanter Parameter P in Form eines oder mehrerer Parameter P und/oder Datensätze 2, Erzeugung von Freigabesignalen 13 und/oder die Auswahl einer oder mehrerer Option 11 insbesondere über die Benutzerschnittstelle 7 und/oder über den Eingabekanal 1.

Im vorliegenden Beispiel wird ein Datensatz 2R, der insbesondere zum einen übergeordnete, personenbezogene Parameter P aufweist und zum anderen ein Datensatz 2A, 2B, der während des Verfahrens bzw. während des Therapieablaufs erhobene Parameter P, wie beispielsweise Vitalparameter, aufweist, derart verarbeitet, dass ein oder mehrere Therapievorschläge bzw. Therapiestationen(en) T₃ ganz oder teilweise, insbesondere als Option 11, für die Weiterbehandlung des Patienten in einer nachfolgenden Phase I-VI ermittelt werden.

Die Therapievorschläge, Therapiestation(en) T₃, hierzu korrespondierende Kenngrößen K und/oder Arbeitsanweisungen bzw. Handlungsempfehlungen können unverändert ganz oder teilweise über die Benutzerschnittstelle 7 ausgegeben werden.

Bevorzugt wird ein mit den verschiedenen Therapievorschlägen bzw. Optionen 11 im Zusammenhang stehender Ressourcenbedarf ermittelt. Der Ressourcenbedarf kann zu einem Parameter P des jeweiligen Datensatzes 2 korrespondieren.

Unter Ressourcen sind in diesem Zusammenhang insbesondere aus therapeutischer Sicht notwendige Gegenstände, Materialien, Geräte zur Untersuchung und/oder Behandlung, entsprechende Räumlichkeiten sowie die Zugänglichkeit zu diesen Räumlichkeiten und/oder medizinisch-therapeutisch tätiges Personal, vorzugsweise für eine Therapiestation T₁, T₂, T₃, T_{N} zu verstehen.

Der Ressourcenbedarf wird beispielsweise durch die Abfrage von Datensätzen 2R aus einer oder mehreren externen Datenbanken 8 ermittelt. Mit den Datensätzen 2R können bestimmte, zu einer oder mehreren Therapiestationen T₁, T₂, T₃, T_{N} korrespondierende Datensätze 2A, 2B, 2C, 2N bzw. darin enthaltenen Parameter P, insbesondere Vitalparameter, in Beziehung gesetzt werden.

Es ergeben sich so beispielsweise bei Vorliegen bestimmter Symptome entsprechende Behandlungsmöglichkeiten in Form von Therapiestationen T₃ und/oder Folgen mehrerer Therapiestationen T₃ und/oder mehrerer alternativen Therapiestationen T₃ bzw. Folgen hiervon als Optionen 11. Diese wiederum korrespondieren vorzugsweise, insbesondere durch Querverweise, zu anderen externen Datenbanken 8, mit entsprechenden Ressourcen.

Auf diese Weise wird ein Parameter P, vorzugsweise des neuen Datensatzes 2C, insbesondere als Ergebnis 9 oder Option 11 ermittelt, der den für die verschiedenen Behandlungsmöglichkeiten, Therapiestationen T₃ bzw. Folgen mehrerer Therapiestationen T₃ o. dgl. erforderliche Ressourcenbedarf repräsentiert.

Ein Ressourcenbedarf oder hierzu korrespondierender Parameter P des Datensatzes 2C kann zusammen mit den Therapiestationen T₃ über die Benutzerschnittstelle 7 ausgegeben werden, so dass einem Benutzer, bei dem es sich insbesondere um einen Arzt handeln kann, eine Übersicht über die ermittelten Optionen 11 bzgl. Therapiemöglichkeiten und die dafür erforderlichen Ressourcen präsentiert wird.

Im weiteren Verlauf kann eine manuelle Eingabe über die Benutzerschnittstelle 7 eine Auswahl bzw. Freigabe durch den Benutzer getroffen werden, vorzugsweise durch das Freigabemittel 12.

Durch ein durch das Freigabemittel 12 übermitteltes Freigabesignal 13 werden daraufhin in einem nachfolgenden Freigabeschritt die ausgewählten Optionen 11 bzw. Therapierstationen T₁, T₂, T₃, T_{N} zur weiteren Ausgabe über die Benutzerschnittstelle 7, insbesondere am Ort der nachfolgenden Phase I-VI und/oder einem einen Patenten zugeordneter Teil der Benutzerschnittstelle 7 freigeben.

Die Freigabe kann alternativ oder zusätzlich vom System S, d. h. beispielsweise durch Übermittlung des Freigabesignals 13 von der Datenverarbeitungseinrichtung 5 an das und/oder durch das Freigabemittel 12, vorzugsweise automatisch, erfolgen.

Vorzugsweise wird eine Ressourcenverfügbarkeit, insbesondere von einem Ressourcenmanagementsystem, abgefragt und/oder ausgelesen, insbesondere in Form des Datensatzes 2R. Die auf diese Weise ermittelte Ressourcenverfügbarkeit wird von der Datenverarbeitungseinrichtung 5 mit den Optionen 11 in Verbindung stehenden Ressourcenbedarf, derart in Beziehung gesetzt, dass aufgrund einer mangelnden Ressourcenverfügbarkeit nicht in Frage kommende Therapiestationen T₁, T₂, T₃, T_{N} verworfen und/oder Optionen 11 mit verfügbaren Ressourcen ausgegeben, hervorgehoben oder auf sonstige Weise ausgewählt werden.

Vorzugsweise erfolgt durch Erzeugung und Übermittlung des Freigabesignals 13 durch das Freigabemittel 12 im nachfolgenden Schritt die Freigabe einer Auswahl der Optionen 11 zur Ausgabe über die Benutzerschnittstelle 7, wobei die Auswahl nur diejenigen Therapiestationen T₁, T₂, T₃, T_{N} als Optionen 11 umfasst, die hinsichtlich der Verfügbarkeit der erforderlichen Ressourcen verfügbar sind.

Im Ergebnis findet vorzugsweise eine Ausgabe über die Benutzerschnittstelle 7 statt, wobei die Ausgabe vorzugsweise wenigstens eine Arbeitsanweisung bzw. Handlungsempfehlung in Form oder basierend auf einem ermittelten Parameter P umfasst.

Die Arbeitsanweisung bzw. Handlungsempfehlung bezieht sich insbesondere auf eine oder mehrere Optionen 11 bzgl. Therapiestationen T₁, T₂, T₃, T_{N} zur Anwendung in der bevorstehenden Phase I-VI. Dementsprechend ist die Benutzerschnittstelle 7 vorzugsweise zur Ausgabe der Therapiestationen T₁, T₂, T₃, T_{N} bzw. der Auswahl möglicher Therapiestationen T₁, T₂, T₃, T_{N} in Form einer Arbeitsanweisung oder Handlungsempfehlung an einem Standort vorgesehen bzw. angeordnet, welcher der nachfolgenden Phase I-VI zugeordnet ist.

Über die Benutzerschnittstelle 7 können in der Folge neue Parameter P eingegeben werden, die als weiterer Datensatz 2 oder in dem Datensatz 2A, 2B, 2C, 2N der aktuellen Therapiestsation T₁, T₂, T₃, T_{N} in der Datenbank 3 gespeichert werden.

Ferner können im Laufe der nun aktuellen Phase I-VI weitere therapiebezogene Parameter P über einen Eingabekanal 1 eingespeist werden.

Da die Datensätze 2A, 2B, die zu Therapiestationen T1, T2 der vorhergehenden Phasen I-VI korrespondieren, vorzugsweise nicht gelöscht werden, stehen diese weiterhin in der Datenbank 3 zur Verfügung, um insbesondere mittels der Datenverarbeitungseinrichtung 5 verarbeitet zu werden.

Die Verarbeitung der Datensätze 2A, 2B mit den Parametern P erfolgt in der nun aktuellen Phase I-VI analog zu den Prozessen der vorhergehenden Phase I-VI. Dabei können Datensätze 2A, 2B insbesondere zu verschiedenen Therapiestationen T1, T2 aus unterschiedlichen Phasen I-VI des Therapieablaufs korrespondieren.

Es steht mit fortschreitendem Therapieablauf eine zunehmende Zahl von Datensätzen 2A, 2B, 2C bzw. Parametern P zur Verfügung, die vorzugsweise jeweils zu unterschiedlichen Therapiestationen T₁, T₂, T₃, T_{N} korrespondieren, um im Hinblick auf eine Ausgabe über die Benutzerschnittstelle 7 verarbeitet zu werden.

Es ist für personenbezogene Parameter P charakteristisch, dass sie dem Therapieablauf als solchem übergeordnet sind. Das bedeutet, dass sie nur mit geringer Wahrscheinlichkeit und insbesondere unkorreliert zum Therapieablauf während des Therapieablaufs geändert, ergänzt oder in anderer Weise beeinflusst werden. Dies ist jedoch nicht vollkommen ausgeschlossen. Es ist daher möglich, dass während des Therapieablaufs, insbesondere beim Übergang von einer Phase I-VI zur nächsten Phase I-VI, weitere personenbezogene Parameter P miteinbezogen werden oder sich die Berücksichtigung der personenbezogenen Parameter P ändert.

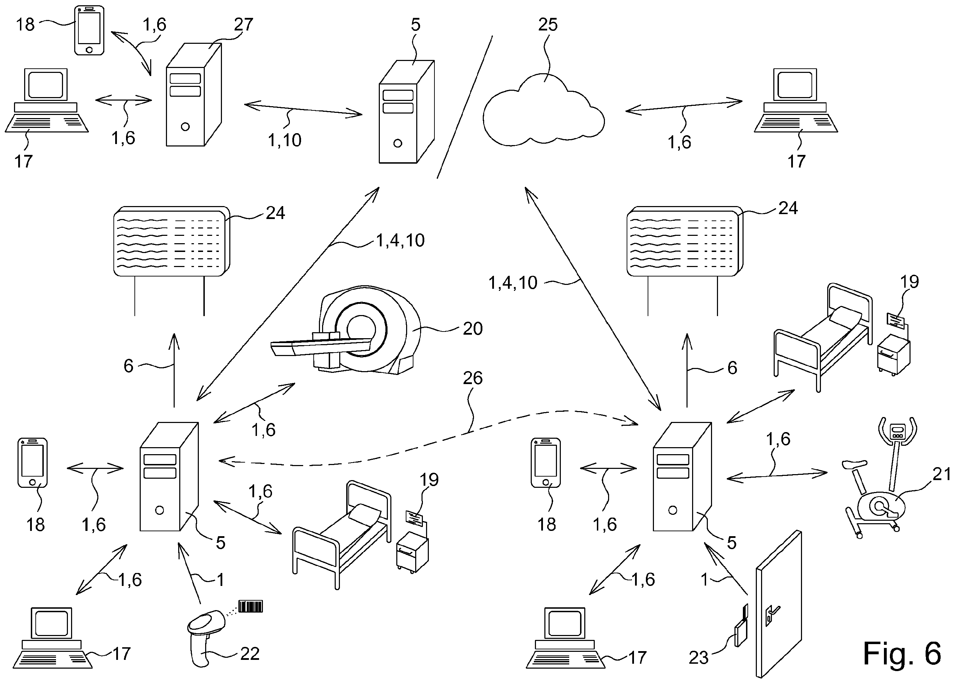

In Fig. 6 ist beispielhaft eine Infrastrukturanordnung wiedergegeben, die zur Implementation des Verfahrens geeignet ist, bzw. dessen Komponenten als Systems zusammenwirken.

Das System S besteht in vorliegender Darstellung aus zwei Untersystemen 15, 16, insbesondere einem ersten Untersystem 15 und einem zweiten Untersystem 16. Jedes der Untersysteme 15, 16 bildet ein Subnetz mit verschiedenen Komponenten, oder weist ein solches auf.

Die Komponenten und die Infrastruktur des Subnetzes eines Untersystems 15, 16 können sich insbesondere an einem Standort einer medizinisch-therapeutischen Einrichtung befinden. Die Untersysteme 15, 16 und deren Subnetze befinden sich jedoch bevorzugt an unterschiedlichen Orten, können jedoch datentechnisch verbunden sein bzw. einen Verbund bilden.

Das Netzwerk kann insbesondere weitere Subnetze aufweisen und, vorzugsweise, zumindest teilweise als drahtloses Netzwerk ausgebildet sein.

Die Untersysteme 15, 16 weisen jeweils eine Datenverarbeitungseinrichtung 5 als zentrales Element auf. Die Datenverarbeitungseinrichtung 5 ist in vorliegender Darstellung als Server ausgeführt, der neben der Datenverarbeitung und/oder der Bereitstellung der Datenbank 3 auch die Bereitstellung wesentlicher Netzwerkfunktionen übernimmt.

Alternativ oder zusätzlich kann auch wenigstens eine zentrale bzw. gemeinsam verwendete Datenverarbeitungseinrichtung 5 für mehrere Subnetze vorgesehen sein. Ferner können die Datenverarbeitungseinrichtungen 5 synchronisiert sein und/oder im Master-Slave-Betrieb arbeiten.

Zu den Teilnehmern des Netzwerks zählen insbesondere Clientrechner 17. Ein solcher Clientrechner 17 kann beispielsweise als Benutzerschnittstelle 7 dienen und somit für die Eingabe und/oder Ausgabe von Datensätzen 2A, 2B bzw. Parametern P, die in den Datensätzen 2A, 2B enthalten oder hierfür bestimmt sein können, vorgesehen sein.

Vorzugsweise kann eine Software auf dem Clientrechner 17 oder auch eine Webanwendung, beispielsweise eine Webseite bzw. ein Online-Portal, die von der Datenverarbeitungseinrichtung 5 bereitgestellt wird, die Funktion der Benutzerschnittstelle 7 übernehmen.

Ferner kann die Benutzerschnittstelle 7 als Mobilgerät 18, insbesondere einer tragbaren Kommunikationseinrichtung, wie einem Smartphone oder einem Tablet-Computer, ausgeführt sein. Ein solches Mobilgerät 18 ist vorzugsweise über ein drahtloses Netzwerk in das Untersystem 15, 16 eingebunden. Dadurch ist es grundsätzlich möglich, von einem beliebigen Ort innerhalb der Reichweite des Drahtlosnetzwerks über die Benutzerschnittstelle 7 auf das System S zuzugreifen, um eine Ausgabe mit einer Arbeitsanweisung abzurufen oder um Parameter manuell über die Benutzerschnittstelle 7 ins System einzugeben und, vorzugsweise, als Datensatz 2 in der Datenbank 3 abzuspeichern.

Ferner kann ein sogenanntes bed side device 19 am Bett des Patienten oder in dessen unmittelbarer Umgebung eine Benutzerschnittstelle 7 aufweisen. Dadurch ist es medizinisch-therapeutischem Personal oder Patienten möglich, am Ort der Therapiestation T₁, T₂, T₃, T_{N}, insbesondere in unmittelbarer Umgebung des Patienten, eine Arbeitsanweisung in Form einer Ausgabe über die Benutzerschnittstelle 7 abzurufen und/oder Parameter P über die Benutzerschnittstelle 7 einzugeben. Dies ist insbesondere von Bedeutung im Bereich der Intensivversorgung, da sich hieraus ein erheblicher Zeitvorteil in der Behandlung eines Patienten ergibt.

Ein solches bed side device 19 ist insbesondere zur manuellen oder automatischen Eingabe von Parametern P, insbesondere von Vitalparametern nach deren Erfassung, ausgebildet. Alternativ oder zusätzlich kann vorgesehen sein, Sensoren zur Erfassung bzw. Messung von Vitalparametern an ein solches bed side device 19 anzuschließen um Parameter P zu generieren bzw. einzuspeisen.

Möglich ist darüber hinaus die Erweiterung bestehender Systeme zur Erfassung und Wiedergabe von Vitalparametern, wie sie beispielsweise im Bereich der Intensivversorgung häufig vorgesehen sind, um Mittel, insbesondere eine entsprechende Hardware und/oder Software, zur Einbindung in das erfindungsgemäße System S. Bestehende Sensoren und Messvorrichtungen für Vitalparameter können in diesem Fall vorzugsweise benutzt werden, um Parameter P für die Datensätze 2A, 2B, 2C, 2N zu generieren. Hierzu können vorhandene Medizingeräte datentechnisch angebunden oder ausgelesen werden.

Möglich ist zudem die direkte Übertragung von Parametern P von einem medizinisch-diagnostischen Gerät 20. Dies betrifft insbesondere medizinischdiagnostische Geräte 20 für bildgebende Verfahren. Erfasste Bilder können in diesem Fall direkt in der Datenbank 3 abgespeichert werden, so dass sie systemweit als Kenngröße für die Freigabe bzw. Auswahl einer oder mehrerer folgender Therapiestationen T₁, T₂, T₃, T_{N} dienen und/oder zur Verfügung stehen.

Alternativ oder zusätzlich zu der Übertragung von Parametern vom medizinisch-diagnostischen Gerät 20 zur Datenverarbeitungseinrichtung 5 bzw. zur Datenbank 3 hin kann auch die umgekehrte Übertragungsrichtung vorgesehen sein. Dadurch lassen sich beispielsweise Parameter P zum medizinisch-diagnostischen Gerät 20 hin übertragen, die relevant für eine mit dem medizinisch-diagnostischen Gerät 20 durchzuführende Untersuchung sind. Diese Parameter P sind vorzugsweise Teil des jeweils zu der Therapiestation T₁, T₂, T₃, T_{N}, zu der das medizinischdiagnostische Gerät 20 zugeordnet ist, korrespondierenden Datensatzes 2A, 2B, 2C

Entsprechendes gilt vorzugsweise für ein therapeutisches Gerät 21, das als Bestandteil des erfindungsgemäßen Systems S ebenfalls in ein entsprechendes Netzwerk eingebunden sein kann. Bei dem therapeutischen Gerät 21 kann es sich insbesondere um ein Mittel zur Mobilisierung eines Patienten handeln, beispielsweise um ein Anti-Schwerkraft-Laufband oder ein BTE-Gerät.

Das vorschlagsgemäße System S kann ein Mittel zur Registrierung und/oder Positionsbestimmung von Gegenständen und/oder Personen aufweisen. In der vorliegenden Darstellung gemäß Fig. 6 handelt es sich dabei insbesondere um einen Barcode-Leser 22, mittels dem entsprechende Markierungen auf Gegenständen wie Medizingeräten an verschiedenen Therapiestationen T₁, T₂, T₃, T_{N} erfasst werden können, so dass stets die aktuelle Position und/oder Eigenschaften der Gegenstände bekannt ist. Dies gilt beispielsweise für die physische Patientenakte oder spezielles, am Standort nicht in großer Zahl vorhandenes, tragbares medizinisches Gerät oder auch für ein entsprechend gekennzeichnetes Patientenbett.

Alternativ oder zusätzlich zu einer Kennzeichnung mit einem Barcode und einem entsprechende Lesegerät als Eingabemittel für entsprechende Parameter P können RFID-Chips und entsprechende Lesegeräte oder Magnetkarten in Verbindung mit einem Magnetkartenleser 23 zu ähnlichen Zwecken eingesetzt werden. Ein Magnetkartenleser 23 eignet sich in besonderer Weise dazu, Personen über einen mit einem Magnetstreifen versehenen Ausweis beim Betreten verschiedener Bereiche der medizinisch-therapeutischen Einrichtung zu erfassen, um bei Bedarf ihren Standort als Parameter P bei einer Datenverarbeitung mittels der Datenverarbeitungseinrichtung 5 miteinbeziehen zu können. Dies kann beispielsweise in Notfallsituationen von Bedeutung sein. Hierzu können jedoch auch andere Identifikationsverfahren verwendet werden.

Soll beispielsweise der verantwortliche Arzt bei einem Notfall hinzugezogen werden, hält sich jedoch in größerer Entfernung von dem Ort des eingetretenen Notfalls auf, so kann vertretungsweise ein anderer Arzt in der Nähe informiert werden, um ohne größere Zeitverzögerung eingreifen zu können. Hierzu kann dem jeweiligen Arzt eine Benutzerschnittstelle 7 zur Verfügung stehen, über die entsprechende Informationen, Handlungsempfehlungen, Arbeitsanweisungen und/oder Therapiestationen T₁, T₂, T₃ bzw. hierzu korrespondierende Parameter P ausgegeben, insbesondere angezeigt werden können.

Ferner stehen über die Benutzerschnittstelle 7 vorzugsweise relevante Daten zum Therapieablauf, insbesondere zu anstehenden Therapiestationen T₁, T₂, T₃, T_{N} des Patienten zur Verfügung. Darüber hinaus können über die Benutzerschnittstelle 7 weitere Anweisungen in das System S eingespeist, am Ort des Notfalls, beispielsweise über das bed side device 19 abgerufen werden können, so dass die folgende Therapiestation T₃ berücksichtigt werden kann.

Die Benutzerschnittstelle 7 kann ferner als eine insbesondere elektronische Schautafel 24 ausgebildet sein. Die Schautafel 24 kann dabei Informationen bezüglich durchzuführender Untersuchungen und/oder Behandlungen bzw. folgender Therapiestationen T₁, T₂, T₃, T_{N} Kenngrößen etc. wiedergeben. Die wiedergegebenen Informationen können darüber hinaus Daten zur Terminierung von Untersuchungen und/oder Behandlungen umfassen, so dass eine über die Benutzerschnittstelle 7 in Form der Schautafel 24 ausgegebene Arbeitsanweisung beispielsweise darin besteht, bestimmte Patienten zu bestimmten Therapiestationen T₁, T₂, T₃, T_{N}, d. h. bestimmten Untersuchungen und/oder Behandlungen, zu überstellen.

Eine solche Schautafel 24 kann beispielsweise in Wartebereichen vor zentralen medizinisch-diagnostischen Geräten 20 in der medizinisch-therapeutischen Einrichtung vorgesehen sein. Alternativ oder zusätzlich ist es auch möglich, dass die Schautafel 24 in ausschließlich für medizinisch-therapeutisches Personal zugänglichen Bereichen vorgesehen ist und Daten über eine Mehrzahl von Patienten, insbesondere von Patienten einer Station bzw. einer organisatorischen Einheit der medizinisch-therapeutischen Einrichtung, ausgibt.

Eine Arbeitsanweisung, die über die Benutzerschnittstelle 7 ausgegeben wird, kann in diesem Fall beispielsweise die Art und Weise und Reihenfolge der Durchführung von Pflegemaßnahmen bei den Patienten einer Station betreffen. Es können daher zu Therapiestationen T₁, T₂, T₃, T_{N} unterschiedlicher Patienten korrespondierende Parameter P durch dieselbe Benutzerschnittstelle 7 ausgegeben, insbesondere chronologisch dargestellt werden.

In Fig. 6 sind die beiden Unternetzwerke 15, 16 jeweils mit einer zentralen Datenverarbeitungseinrichtung 5 verbunden. Durch eine mögliche Verbindung der zentralen Datenverarbeitungseinrichtung 5 mit wenigstens einem der Unternetze 15, 16 ist es möglich, dass die zentrale Datenverarbeitungseinrichtung 5 sich an einem geografisch beabstandeten Ort befindet.

Bei einer bevorzugten Ausgestaltung des Systems S ist die zentrale Datenverarbeitungseinrichtung 5 am Standort einer medizinisch-therapeutischen Einrichtung angeordnet. Die medizinisch-therapeutische Einrichtung mit der zentralen Datenverarbeitungseinrichtung 5 kann dabei insbesondere ein eigenes Untersystem 15, 16 aufweisen.

Dabei kann zusätzlich zu der zentralen Datenverarbeitungseinrichtung 5 wenigstens eine weitere, dem Untersystem 15, 16 der medizinisch-therapeutischen Einrichtung mit der zentralen Datenverarbeitungseinrichtung 5 zugeordnete Datenverarbeitungseinrichtung 5 vorgesehen sein. Möglich ist jedoch ebenso, dass eine Datenverarbeitungseinrichtung 5 eines Untersystems 15, 16, insbesondere des ersten Untersystems 15, die Funktion einer zentralen Datenverarbeitungseinrichtung 5 für das gesamte System S übernimmt.

Die zentrale Datenverarbeitungseinrichtung 5 kann die Verarbeitung von Datensätzen 2 für das gesamte System S leisten. Die zentrale Datenverarbeitungseinrichtung 5 ist dazu insbesondere über einen Eingabekanal 1, einen Auslesekanal 4 und/oder einen internen Ausgabekanal 10 mit wenigstens einem Untersystem 15, 16, insbesondere mit wenigstens einer Datenverarbeitungseinrichtung 5 wenigstens eines Untersystems 15, 16, verbunden.

Vorzugsweise weist die zentrale Datenverarbeitungseinrichtung 5 Speichermittel für die Datenbank 3 auf, die auf diese Weise als zentralen Datenbank 3 für das System S fungiert. Alternativ oder zusätzlich kann auch vorgesehen sein, dass ein oder mehrere Datensätze 2A, 2B, 2C bzw. Teile der Datenbank 3 in einem dezentralen Cloudspeicher 25 gespeichert sind.

Die Datenübertragung, insbesondere die Übertragung von Datensätzen 2A, 2B, 2C von der und/oder zur Datenbank 3 und/oder Datenverarbeitung mittels einer Datenverarbeitungseinrichtung 5, erfolgt vorzugsweise zumindest im Wesentlichen über die zentrale Datenverarbeitungseinrichtung 5. Darüberhinaus kann jedoch ein Kommunikationskanal 26 vorgesehen sein, der wenigstens zwei Untersysteme 15, 16 direkt miteinander verbindet.

Der Kommunikationskanal 26 kann zur Übertragung von Datensätzen 2A, 2B, 2C, d. h. als Eingabekanal 1, Auslesekanal 4, Ausgabekanal 6 und/oder interner Ausgabekanal 10, dienen. Es ist darüberhinaus auch möglich, dass Nutzer bzw. Benutzer des Systems S, insbesondere unter Verwendung der Benutzerschnittstelle 7, über den Kommunikationskanal 26 miteinander kommunizieren können.

Es versteht sich, dass ein solcher Nachrichtenaustausch ebenso über die zentrale Datenverarbeitungseinrichtung 5 erfolgen kann.

Insbesondere zur Bereitstellung einer Webseite bzw. eines Online-Portals in der Funktion einer Benutzerschnittstelle 7, auf die von einem nahezu beliebigen Standort aus zugegriffen werden kann das System S einen Webserver 27 aufweisen. Der Webserver 27 stellt vorzugsweise ein Mittel für den Zugang zum erfindungsgemäßen System S von außen dar. Insbesondere über das Internet kann mit einem Clientrechner 17, einem internetfähigen Mobilgerät 18 oder vergleichbaren Kommunikationsmitteln auf den Webserver 27 als Benutzerschnittstelle 7 auf das System S zugegriffen werden.

Der Zugriff auf das System S über eine Webanwendung der vorgenannten Art, d. h. über den Webserver 27, ermöglicht es beispielsweise Patienten, von ihrem Wohnort aus selbstständig Parameter P bzw. Datensätze 2 in die Datenbank 3 einzuspeisen, indem sie beispielsweise selbstgemessene Vitalparameter über das Online-Portal als Benutzerschnittstelle 7 eingeben. Darüber hinaus kann beispielsweise ein niedergelassener Arzt über eine vom Webserver 27 bereitgestellte Webseite als Benutzerschnittstelle 7 auf die in der Datenbank 3 gespeicherte Datensätze 2 zugreifen, ohne sich physisch an einen der Standorte der Komponenten des Systems S zu begeben.

Die Webseite bzw. das Online-Portal weist aus Datenschutzgründen vorzugsweise ein Mittel zur Registrierung eines oder mehrerer Benutzer auf. Hierdurch kann insbesondere durch die Eingabe von Log-In-Daten accountweise Zugang zum System S gewährt werden. Dabei können unterschiedliche Accounttypen vorgesehen sein, die insbesondere über unterschiedliche Zugangsbefugnisse verfügen.

Der Webserver 27 kann physikalisch eigenständig ausgebildet sein und ist in diesem Fall insbesondere über einen Eingabekanal 1 und/oder einen internen Ausgabekanal 10 mit einer, insbesondere zentralen, Datenverarbeitungseinrichtung 5 und/oder Datenbank 3 verbunden. Alternativ kann der Webserver 27 beispielsweise auch in die physikalische Ausgestaltung der zentralen Datenverarbeitungseinrichtung 5 integriert sein.

**Bezugszeichenliste:**

| | | | |
|---|---|---|---|
| 1 | Eingabekanal | T₂ | Therapiestation |
| 2 | Datensatz | T₃ | Therapiestation |
| 2A | Erster Datensatz | T_{N} | Therapiestation |
| 2B | Zweiter Datensatz | | |
| 2C | Dritter Datensatz | P | Parameter |
| 2N | N-ter Datensatz | P_{A1} | Parameter |
| 2R | Ressourcendatensatz | P_{A2} | Parameter |
| 3 | Datenbank | P_{AM} | Parameter |
| 4 | Auslesekanal | P_{AE} | Ergebnisparameter |
| 4A | Rückkanal | P_{B1} | Parameter |
| 5 | Datenverarbeitungseinrichtung | P_{B2} | Parameter |
| 6 | Ausgabekanal | P_{BM} | Parameter |
| 7 | Benutzerschnittstelle | P_{BE} | Ergebnisparameter |
| 8 | externe Datenbank | P_{N1} | Parameter |
| 9 | Ergebnis | P_{N2} | Parameter |
| 10 | interner Ausgabekanal | P_{NM} | Parameter |
| 11 | Option | P_{NE} | Ergebnisparameter |
| 12 | Freigabemittel | | |
| 13 | Freigabesignal | | |
| 14 | Schnittstelle | A | Ausgabe |
| 15 | erstes Untersystem | I | ampelartige Anzeige |
| 16 | zweites Untersystem | K | Kenngröße |
| 17 | Clientrechner | R | Ressource |
| 18 | Mobilgerät | S | System |
| 19 | bed side device | | |
| 20 | medizinisch-diagnostisches Gerät | | |
| 21 | therapeutisches Gerät | | |
| 22 | Barcode-Leser | | |
| 23 | Magnetkartenleser | | |
| 24 | Schautafel | | |
| 25 | Cloudspeicher | | |
| 26 | Kommunikationskanal | | |
| 27 | Webserver | | |
| | | | |
| T₁ | Therapiestation | | |

## Patentansprüche

1. System (S) zur Unterstützung bei einem Therapieablauf,
wobei das System (S) wenigstens eine Datenbank (3) zur Speicherung von Datensätzen (2) aufweist,
wobei das System (S) wenigstens eine, insbesondere zentrale, Datenverarbeitungseinrichtung (5) zur Verarbeitung der Datensätzen (2) aufweist, und
wobei das System (S) wenigstens eine Benutzerschnittstelle (7) zur Eingabe und/oder Ausgabe von Datensätzen (2) aufweist,
wobei das System (S) dazu ausgebildet ist, eine Ausgabe durch die Benutzerschnittstelle auf Basis mindestens zweier, zu unterschiedlichen Therapiestationen (T₁, T₂), korrespondierender, patientenspezifische und/oder therapierelevante Parameter aufweisender Datensätze (2) erfolgt, wobei die Ausgabe eine, vorzugsweise eine therapeutische Maßnahme, insbesondere eine Medikation, eine medizinische Untersuchung und/oder eine Rehabilitationsmaßnahme betreffende, Arbeitsanweisung und/oder Handlungsempfehlung an einen Benutzer umfasst.

2. System nach Anspruch 1, **dadurch gekennzeichnet, dass** die Benutzerschnittstelle (7) eine insbesondere elektronische Schautafel (24) und/oder ein Mittel zur akustischen Signalisierung aufweist; und/oder
dass die Benutzerschnittstelle (7) zur insbesondere standortunabhängigen Ein- und/oder Ausgabe von Datensätzen (2), vorzugsweise als Software für ein Mobilgerät (18) und/oder als Webseite, ausgestaltet ist.

3. System nach Anspruch 1 oder 2, **dadurch gekennzeichnet, dass** wenigstens ein Mittel zur Registrierung und/oder Positionsbestimmung von Gegenständen und/oder Personen vorgesehen ist.

4. Verwendung eines Systems (S) nach einem der vorhergehenden Ansprüche zur Benutzerführung und/oder Ermittlung einer Therapiestation (T₁, T₂) im Bereich der Akutversorgung, Pflege und/oder Rehabilitationsbehandlung von Patienten.

5. Verfahren zur informationstechnischen Unterstützung bei einem Therapieablauf,
wobei eine Ausgabe durch eine Benutzerschnittstelle (7) auf Basis mindestens zweier, zu unterschiedlichen Therapiestationen (T₁, T₂) korrespondierender, patientenspezifische und/oder therapierelevante Parameter (P) aufweisender Datensätze (2A, 2B) erfolgt.

6. Verfahren nach Anspruch 5, **dadurch gekennzeichnet, dass** auf die zu unterschiedlichen Therapiestationen (T₁, T₂) korrespondierenden Datensätze (2A, 2B) bzw. deren Parameter (P) gemeinsam verarbeitet werden und/oder dass auf die zu unterschiedlichen Therapiestationen (T₁, T₂) korrespondierenden Datensätze (2A, 2B) bzw. deren Parameter (P) ein Algorithmus, angewendet wird, um wenigstens einen neuen Datensatz (2C) und/oder eine Arbeitsanweisung und/oder Handlungsempfehlung zu erzeugen, vorzugsweise wobei
der neue Datensatz (2C) und/oder die Arbeitsanweisung und/oder Handlungsempfehlung zu einer weiteren, insbesondere einer dritten, Therapiestation (T₃) korrespondiert; und/oder
anhand des neuen Datensatzes (2C) wenigstens eine Eigenschaft, insbesondere ein Ressourcenbedarf, der weiteren Therapiestation (T₃) ermittelt wird; und/oder
wenigstens eine Maßnahme zur Vorbereitung einer weiteren, insbesondere einer dritten, Therapiestation (T₃), bevorzugt automatisch, initiiert wird; und/oder die zu unterschiedlichen Therapiestationen (T₁, T₂) korrespondierenden Datensätze (2C) oder Parameter (P) dieser zu unterschiedlichen Therapiestationen (T₁, T₂) korrespondierenden Datensätze (2A, 2B) miteinander verglichen oder verrechnet werden und mit oder aus dem Ergebnis dieses Vergleichs oder dieser Verrechnung der neue Datensatz (2C) und/oder die Arbeitsanweisung und/oder Handlungsempfehlung erzeugt bzw. eine weitere Therapiestation (T₃) ermittelt wird
und/oder wobei die Ausgabe eine, vorzugsweise eine therapeutische Maßnahme, insbesondere Medikation, medizinische Untersuchung und/oder Rehabilitationsmaßnahme betreffende, Arbeitsanweisung und/oder Handlungsempfehlung an einen Benutzer umfasst.

7. Verfahren nach Anspruch 5 oder 6, **dadurch gekennzeichnet, dass** ein oder mehrere der Parameter:
Vitalparameter des Patienten sind, die zu Vitalfunktionen des Patienten korrespondierende Werte oder Verläufe repräsentieren; und/oder
mittels Sensoren, insbesondere Sensoren zur Messung von Vitalparametern, erfasst werden; und/oder
manuell über die Benutzerschnittstelle (7) registriert werden; und/oder
von einem medizinisch-diagnostischen Gerät (20) und/oder von einem Ressourcenmanagementsystem ausgelesen werden; und/oder
in einer Datenbank (3), insbesondere zentral, gespeichert werden oder sind.

8. Verfahren nach einem de Ansprüche 5 bis 7, **dadurch gekennzeichnet, dass** eine Freigabe der Arbeitsanweisung und/oder der Handlungsempfehlung mittels eines Freigabesignals (13) insbesondere an ein Freigabemittel (12) erfolgt, vorzugsweise wobei das Freigabesignal (13) auf die Ausgabe der Arbeitsanweisung und/oder der Handlungsempfehlung hin mittels, insbesondere durch oder mit einem Sensor, der Benutzerschnittstelle (7) erzeugt wird.

9. Verfahren nach einem der Ansprüche 5 bis 8, **dadurch gekennzeichnet, dass** eine Kenngröße, insbesondere ein Ressourcenbedarf, mit mehreren, zu unterschiedlichen Therapiestationen (T₁, T₂) korrespondierenden Datensätze (2A, 2B) ermittelt wird, vorzugsweise wobei die Kenngröße über die Benutzerschnittstelle (7), wiedergegeben wird; und/oder wobei die Kenngröße über die Benutzerschnittstelle (7) mit wenigstens einem Datensatz (2) eines Ressourcenmanagementsystems in Bezug gesetzt wird.

10. Verfahren nach einem der Ansprüche 5 bis 9, **dadurch gekennzeichnet, dass** das eine zukünftige Entwicklung der Kenngröße, vorzugsweise anhand eines oder mehrerer Datensätze (2A, 2B), simuliert oder prognostiziert wird.

11. Computerprogrammprodukt, insbesondere computerlesbares Speichermedium, aufweisend Programmcodemittel, die dazu ausgebildet sind, wenn sie auf einem Computer oder Prozessor ausgeführt werden, das Verfahren gemäß einem der Patentansprüche 5 bis 10 auszuführen.
